# EUROPÄISCHE PATENTSCHRIFT

(11) **EP 1 692 250 B1**
(45) Veröffentlichungstag und Bekanntmachung des Hinweises auf die Patenterteilung: **29.07.2009**
(21) Anmeldenummer: 04803165.2
(22) Anmeldetag: 17.11.2004
(51) Int. Cl.: C11D 1/66, C11D 1/72, C11D 3/00, A61K 31/00

(54) **ADHÄSIONSHEMMUNG VON MIKROORGANISMEN DURCH NICHTIONISCHE TENSIDE**
ADHESION INHIBITION OF MICRO ORGANISMS BY NON-IONIC SURFACTANTS
INHIBITION DE L'ADHERENCE DE MICRO-ORGANISMES AU MOYEN DE TENSIOACTIFS NON-IONIQUES

(30) Priorität: 13.12.2003 DE 10358534
(43) Veröffentlichungstag der Anmeldung: 23.08.2006
(73) Patentinhaber: Henkel AG & Co. KGaA, 40589 Düsseldorf (DE)
(72) Erfinder: WEIDE, Mirko, 40223 Düsseldorf (DE); FREY, Stefan, 67146 Deidesheim (DE); STODT, Jürgen, 41466 Neuss (DE); BOLTE, Andreas, 40627 Düsseldorf (DE); BREVES, Roland, 40822 Mettmann (DE); GERKE, Thomas, 41468 Neuss (DE)
(86) Internationale Anmeldenummer: PCT/EP2004/013038
(87) Internationale Veröffentlichungsnummer: WO 2005/056741

(56) Entgegenhaltungen:
- EP-A- 0 227 108
- EP-A- 0 286 008
- EP-A- 0 341 151
- EP-A- 1 229 091
- WO-A-88/06038
- WO-A-95/15182
- WO-A-97/04768
- WO-A1-00/05183
- WO-A1-96/26783
- WO-A1-97/24290
- AT-B- 395 415
- DE-A- 2 031 109
- DE-A1- 4 301 553
- US-A1- 2002 073 667
- US-B1- 6 235 124
- DATABASE WPI Section Ch, Week 200504 Derwent Publications Ltd., London, GB; Class A97, AN 2005-033862 XP002322055 & JP 2004 346124 A (HOSOKAWA T) 9. Dezember 2004 (2004-12-09)

## Beschreibung

Die Erfindung betrifft die Verwendung von nichtionischen Tensiden zur Verminderung der Anhaftung von Mikroorganismen an Oberflächen sowie Zusammensetzungen, Zubereitungen und Materialien, die diese Stoffe enthalten oder mit diesen Stoffen beschichtet sind.

In unterschiedlichsten Bereichen besteht ein Bedarf, Mittel zur Verfügung zu haben, die die Adhäsion von Mikroorganismen verhindern.

So befinden sich etwa im Haushalt Schimmelpilze an verschiedensten Stellen, bspw. in der Küche oder in feuchten Räumen, wie z.B. im Badezimmer. Schimmelpilze verursachen erhebliche Probleme dadurch, dass die von ihnen in die Raumluft abgegebenen Sporen häufig allergieerzeugend sind. Eine Bekämpfung solcher Pilze mit bioziden Wirkstoffen geht mit einem erhöhten Risiko der Resistenzbildung einher, so dass nach einiger Zeit neue antimikrobielle Substanzen gefunden werden müssen, die gegen diese resistent gewordenen Mikroorganismen wirken. Biozide sind außerdem ökologisch und toxikologisch nicht immer unbedenklich.

Des weiteren werden empfindliche Textilien, wie z. B. Seide oder Mikrofaser, immer häufiger zu Kleidungsstücken verarbeitet, die nur bei 30 oder 40 °C gewaschen werden können. Dadurch werden Pilze, wie beispielsweise die humanpathogene Candida albicans, nicht abgetötet. Insbesondere nach einer Pilzinfektion kann es durch solche auf Kleidungsstücken haftenden, nicht abgetöteten Pilze zu einer Reinfektion kommen.

Weiterhin erkranken Gebissträger häufig an einer oralen Candidose (Soor). An der Oberfläche der Prothese haftende Pilzzellen können bei Kontakt die Schleimhäute besiedeln, die durch Druckstellen oft vorgeschädigt sind.

Um die Reinfektion durch an der Kleidung oder an Kunststoffoberflächen haftenden Mikroorganismen zu verhindern, wurden bisher antimikrobielle Substanzen eingesetzt, die entweder das Wachstum der Mikroorganismen hemmen (Biostatika) oder diese abtöten (Biozide). Nachteilig ist daran, dass solche z. B. in Wasch- und Reinigungsmitteln verwendeten Biozide oder Biostatika die Abwässer belasten und somit auch die mikrobiellen Klärstufen in den Kläranlagen in ihrer Funktion beeinträchtigen. Zudem wird der Selektionsdruck auf die Mikroorganismen zur Resistenzbildung stark erhöht, so dass nach einiger Zeit neue antimikrobielle Substanzen gefunden werden müssen, die gegen diese resistent gewordenen Mikroorganismen wirken.

Darüber hinaus kann die Verminderung der Anhaftung durch den verringerten Kontakt des menschlichen Körpers mit den Mikroorganismenzellen, beispielsweise der Atemwege mit Schimmelpilzsporen, auch zu einer Verminderung des allergieauslösenden Potentials führen.

Aufgabe der vorliegenden Erfindung ist es daher, gezielt Mikroorganismen von Oberflächen zu entfernen, ohne diese Oberflächen oder die Abwässer mit bioziden und/oder biostatischen Wirkstoffen zu belasten.

Diese Aufgabe wird gelöst durch die Verwendung von nichtionischen Tensiden zur Verminderung der Anhaftung von Mikroorganismen an Oberflächen.

Von Polyethylenglykol-Gruppen ist bereits bekannt, dass sie die Adsorption an bestimmte Oberflächen verhindern können, wenn sie kovalent an der betreffenden Oberfläche fixiert werden. Jedoch sind die beschriebenen Verfahren zur Fixierung der Polyethylenglykol-Gruppen sehr aufwendig und zudem bislang auf kommerziell uninteressante Oberflächen beschränkt und/oder in der Praxis schwer umsetzbar. So beschreiben Du et al. die Beschichtung von Glasoberflächen mit Lipidschichten und das anschließende Einbringen von Polyethylenglykol-modifizierten Fetten in die Lipidschichten, um hierdurch die Adsorption von bestimmten Proteinen und Blut-Zellen zu verhindern (Biochimica et Biophysica Acta 1326, 236-248 (1997)). Polyethylenglykol-Gruppen wurden ebenfalls mittels sich selbst organisierenden Lipid-Einzelschichten auf Substratoberflächen aufgebracht. In diesem Zusammenhang wird die Beschichtung von Silber und Gold-Oberflächen mit n-Alkanthiolen, die Oligoethylenglykol-Einheiten tragen, zur Verhinderung der Protein-Adsorption beschrieben (Harder et al. (1998) J. Phys. Chem. B 102, 426-436; Prime et al. (1993) J. Am. Chem. Soc. 115, 10714-10721). Cunliffe et al. (1999; Appl Environ Microbiol. 65(11):4995-5002) beschreiben die Beschichtung von Silicat-Oberflächen mit funktionellen Gruppen, die Aminogruppen enthalten, und das anschließende Koppeln von Polyethylenglykol enthaltenden Gruppen an diese funktionellen Gruppen, um hierdurch die Adsorption von Bakterien und Proteinen zu verhindern. Die genannten Verfahren sind sehr aufwendig und für die Praxis nur bedingt geeignet. Außerdem ist die Durchführung der beschriebenen Verfahren auf Oberflächen beschränkt, die kommerziell nur von geringem Interesse sind. So können mit den beschriebenen Verfahren insbesondere keine der zuvor genannten kommerziell interessanten Oberflächen, wie Kunststoff und Textil-Oberflächen, gegen Adhäsion von Mikroorganismen ausgerüstet werden.

Überraschenderweise wurde nun gefunden, dass auf einfache Weise durch nichtionische Tenside die Adhäsion von Mikroorganismen an Oberflächen vermindert werden kann, ohne dass diese an der Oberfläche kovalent fixiert werden müssen. Dies kann etwa dadurch erreicht werden, dass sie in einen Reiniger oder in ein Ausrüstungsmittel eingebracht werden, mit dem die betreffende Oberfläche behandelt wird. In einer besonders bevorzugten Ausführungsform werden die nichtionischen Tenside jedoch in das Material eingebracht und/oder eingearbeitet, dessen Oberfläche vor der Adhäsion geschützt werden soll.

Der Einsatz von nichtionischen Tensiden in Reinigungsmitteln, in Entfettungsmitteln, in pharmazeutischen Zubereitungen, in Mitteln zur oralen Applikation sowie zur Ausrüstung von Materialien ist im Allgemeinen bereits beschrieben.

So wird in US 6,235,124 die Verwendung einer Lösung enthaltend Natriumperborat und ein nichtionisches Tensid zur Entfernung eines Schimmelpilzes von Oberflächen auf einem Flugzeug offenbart. Allerdings wird hier nicht die Anhaftung des Schimmelpilzes an Oberflächen vermindert, sondern der Schimmelpilz wird durch das Natriumperborat in der Lösung zerstört und anschließend mechanisch entfernt, wobei das nichtionische Tensid lediglich aufgrund seiner oberflächenaktiven Eigenschaften in der verwendeten Lösung enthalten ist.

In DE 4301553 wird ein Entfettungsmittel zur Behandlung von Häuten, Blößen, Fellen und Ledern offenbart, das nichtionische Emulgatoren enthält.

In WO 88/06038 und WO 95/15182 wird die Verwendung von Copolymeren aus Polyoxyethylen- und Polyoxypropylen-Einheiten als antimikrobiell wirksame Substanzen zur Bekämpfung von Infektionen offenbart.

In WO 97/04768 wird die Verwendung von Vesikeln aus nichtionischen Tensiden zur Herstellung eines Agens zur Behandlung von Krankheitszuständen, die auf eine erhöhte Konzentration an Cytokinen zurückzuführen sind, offenbart.

In EP 0227108 werden Mittel zur oralen Applikation beschrieben, die Berberin enthalten, wobei zur Unterdrückung des bitteren Geschmacks des Berberins zusätzlich nichtionische Tenside in den Mitteln enthalten sind.

In EP 1229091 wird die Verwendung von Fluorpolymeren zur Beschichtung von Oberflächen offenbart, wobei die Fluorpolymer-haltige Zusammensetzung zur Stabilisierung der Fluorpolymere nichtionische Tenside enthalten kann.

In EP 0341151 werden Membranen offenbart, die mit nichtionischen Tensiden beschichtet sind.

In WO 96/26783 werden Filtermedien offenbart, die unter anderen mit nichtionischen Tensiden beschichtet sind.

In AT 395415 B wird die Verwendung von Emulsionen enthaltend nicht-ionogene Emulgatoren zur Herstellung Kohlenwasserstoff-absorbierender Betone bzw. Betonsteine offenbart. Als nicht-ionogene Emulgatoren werden aromatische Ethylenoxidaddukte, insbesondere Kresoladdukte genannt.

In EP 0286008 wird die Verwendung von kationischen Kunststoffdispersionen zum Imprägnieren und Grundieren von saugfähigen Substraten offenbart.

In DE 2031109 werden Zusatzmittel zur Modifizierung von Branntkalk offenbart, die unter anderem nichtionische Flockungsmittel enthalten kann. Als Beispiel für nichtionogene Flockungsmittel werden Polyacrylamide offenbart.

In WO 00/0583 wird eine Zubereitung offenbart, die Calcit- oder Silikat-haltigen Konstruktionsmaterialien erhöhte Resistenz gegenüber Schäden durch Wasser, Öl, Fett und Wetter verleiht, wobei die Zubereitung eine Lösung, Emulsion oder Dispersion eines Copolymers, das fluorierte, kationische und nichtionische Gruppen trägt, Wasser sowie ein Penetrationshilfsmittel enthält, wobei es sich bei dem Penetrationshilfsmittel unter anderem auch um nichtionische Tenside handeln kann. Zum Schutz der Materialien wird die Zubereitung auf die zu behandelnden Materialien aufgebracht.

In US 2002/0073667 wird ein Filterelement offenbart, das ein Blatt-artiges Substrat umfasst, das zumindest teilweise durch eine Schicht bedeckt ist, wobei diese Schicht eine polymere dünne Faser umfasst, wobei als Additiv unter anderem ein Mittel zugegen sein kann, das nichtionische Tenside enthält.

In keinem der zuvor genannten Dokumente ist jedoch die Verwendung von nichtionischen Tensiden zur Verminderung der Adhäsion von Mikroorganismen an Oberflächen offenbart.

Weiterhin wird In WO 97/24290 ein Verfahren zur Kontrolle des Biofouling auf submersiven Oberflächen offenbart, bei dem fluorierte Tenside zum Einssatz kommen, wobei es sich bei den fluorierten Tensiden auch um nichtionische Tenside handeln kann. Insbesondere wird hierbei offenbart, dass die fluorierten Tenside dazu dienen, die Adhäsion von Bakterien an diese submersiven Oberflächen zu inhibierend. Die Applikation der fluorierten Tenside erfolgt hierbei dadurch, dass die fluorierten Tenside in das umgebende Medium gegeben oder auf die vor Adhäsion zu schützende Oberfläche aufgetragen werden. Allerdings kann man diesem Dokument nicht entnehmen, dass auch nicht-fluorierte nichtionische Tenside wirksam gegenüber der Adhäsion von Bakterien wären. Weiterhin kann man diesem Dokument ebenfalls nicht entnehmen, dass eine anti-adhäsive Ausrüstung dadurch erreicht werden könnte, dass man die anti-adhäsiven Substanzen in die vor Adhäsion zu schützenden Materialien einarbeitet.

Gegenstand der vorliegenden Erfindung ist daher die Verwendung von nichtionischen Tensiden zur Verminderung der Anhaftung von Mikroorganismen an Oberflächen sowie ein Verfahren zur Verminderung der Adhäsion von Mikroorganismen an Oberflächen, dadurch gekennzeichnet, dass die nichtionischen Tenside in die Materialien eingebracht werden oder in diese eingearbeitet sind, wobei das Einbringen vorzugsweise nicht kovalent erfolgt.

Gegenstand der vorliegenden Erfindung ist daher weiterhin die Verwendung von nichtionischen Tensiden zur Verminderung der Anhaftung von Mikroorganismen an Oberflächen sowie ein Verfahren zur Verminderung der Adhäsion von Mikroorganismen an Oberflächen, dadurch gekennzeichnet, dass die nichtionischen Tenside in die Materialien eingebracht werden und/oder auf die Materialien aufgebracht werden, wobei das Aufbringen und/oder Einbringen vorzugsweise nicht-kovalent erfolgt, wobei die nichtionischen Tenside ausgewählt sind aus:
a) Anlagerungsprodukten von 5 bis 15 Ethylenoxid-Einheiten an C6-C12-Alkylphenole,
b) Anlagerungsprodukten von 10 bis 22 Ethylenoxid-Einheiten an Fettalkohole mit 10 bis 22 C-Atomen,
c) Anlagerungsprodukten von zwischen 5 und 11 Ethylenoxid-Einheiten an Fettalkohole mit 10 bis 20 C-Atomen,
d) Anlagerungsprodukten von zwischen 8 und 12 Ethylenoxid-Einheiten an Fettalkohole mit 18 und 26 C-Atomen,
e) Anlagerungsprodukten von zwischen 3 und 7 Ethylenoxid-Einheiten und zwischen 2 und 6 Propylenoxid-Einheiten an Fettalkohole mit zwischen 10 und 16 C-Atomen.

Unter Mikroorganismen sind insbesondere Bakterien, Pilze sowie Viren und Algen zu verstehen. Dies schließt bakterielle Endo- oder Exosporen sowie Sporen, die als Fortpflanzungsstrukturen bei Pilzen dienen, mit ein.

Unter Verminderung der Anhaftung ist eine signifikante Reduktion der Zahl der anhaftenden Mikroorganismenzellen zu verstehen. Dabei wird die Anhaftung vorzugsweise um mehr als 20 oder 40 %, besonders bevorzugt um mehr als 60 oder 80 %, insbesondere um mehr als 90 oder 95 %, in Bezug zu einer unbehandelten Vergleichsprobe, vermindert. Idealerweise wird die Anhaftung vollständig oder annähernd vollständig verhindert.

Gemäß einer bevorzugten Ausführungsform werden die nichtionischen Tenside in solchen Endkonzentrationen eingesetzt, dass sie nicht biozid oder biostatisch wirken. Ein besonderer Vorteil dieser Ausführungsform ist, dass das Risiko einer Resistenzbildung gegenüber den verwendeten Stoffen gering ist, da die Mikroorganismen weder abgetötet werden noch ihr Wachstum gehemmt wird. Die Konzentrationen, bei denen noch keine Hemmung des Wachstums vorliegt, sowie die minimalen Hemmkonzentrationen selbst können in dem Fachmann bekannter Weise einfach bestimmt werden. Experimentell konnte nachgewiesen werden, dass erfindungsgemäß bevorzugte nichtionische Tenside selbst bei Einsatz in relativ hohen Konzentrationen keine fungizide Wirkung zeigen.

Darüber hinaus sind die meisten nichtionischen Tenside auch aus toxikologischer Sicht, soweit nach heutigem Stand bekannt, unbedenklich.

Ein weiterer Vorteil der Erfindung ist es, dass einige nichtionische Tenside, auch im Vergleich zu herkömmlichen Bioziden oder Biostatika, bereits in geringen Endkonzentrationen wirksam sind, so dass nur wenig Substanz verwendet werden muss.

Weiterer Gegenstand der vorliegenden Erfindung sind Baustoffe und Bauhilfsstoffe ausgewählt aus Klebe-, Dichtungs- und Spachtelmassen, die nichtionische Tenside enthalten und/oder mit nichtionischen Tensiden beschichtet bzw. ausgerüstet sind, wobei es sich vorzugsweise um alkoxylierte, vor allem um ethoxylierte und/oder propoxylierte Tenside handelt, wobei ein Ethoxylierungsgrad von 5 bis 15 bevorzugt ist, und wobei die nichtionischen Tenside auch fluoriert sein können.

Gemäß einer besonders bevorzugten Ausführungsform wird die Anhaftung von Mikroorganismen an Filtermedien, Klebstoffe, Baustoffe und/oder Bauhilfsstoffe vermindert.

Nach einer weiteren bevorzugten Ausführungsform wird die Anhaftung von Mikroorganismen auf den Oberflächen, die häufig in Kontakt mit dem menschlichen Körper kommen, vermindert. Dabei sind insbesondere abiotische, technische (bzw. technisch hergestellte) Oberflächen gemeint. Im Sinne dieser besonderen Ausführungsform sind daher menschliches oder tierisches Gewebe nicht darunter zu verstehen.

Gemäß einer weiteren bevorzugten Ausführungsform wird die Anhaftung von Mikroorganismen an solchen Oberflächen wie Textilien, Keramiken, Metallen und/oder Kunststoffen vermindert. Insbesondere handelt es sich dabei um Wäsche, Sanitäreinrichtungen, Bodenbeläge, Schuhe, Leder, aus Gummi hergestellte Gebrauchsgegenstände, Prothesen oder Zahnersatz.

Die nichtionischen Tenside werden bei den genannten Anwendungen vorzugsweise auf das Material aufgebracht oder in das Material eingebracht bzw. eingearbeitet, ohne kovalent an der Oberfläche fixiert zu werden.

Die Verminderung der Anhaftung an Textilien oder Kunststoffoberflächen vermindert das Risiko einer Reinfektion der befallenen Körperbereiche. Die Verminderung der Anhaftung von Mikroorganismen an Keramiken, Kunststoffen oder Metallen, insbesondere an Prothesen oder Zahnersatz, verringert das Infektions- bzw. Reinfektionsrisiko, ohne die Haut, die Schleimhäute oder die Abwässer mit biozid oder biostatisch bzw. virostatisch wirkenden Substanzen zu belasten. Ebenso können Katheter sowie andere aus Kunststoff oder Metallen hergestellte medizinische Geräte und/oder Prothesen durch die Verwendung nichtionischer Tenside beispielsweise in Spülungen oder Reinigungsmitteln von der Anhaftung befreit werden.

Zahnersatz, insbesondere Gebisse, können durch die Verwendung nichtionischer Tenside in Mund-, Zahn- und/oder Zahnprothesenpflegeprodukten wirksam, einfach und ohne Belastung der behandelten Oberfläche mit stark biozid wirkenden, möglicherweise sogar bedingt toxischen Substanzen von der Mikroorganismenanhaftung gereinigt werden.

### Nichtionische Tenside

Als nichtionische Tenside werden vorzugsweise alkoxylierte, vorteilhafterweise ethoxylierte oder propoxylierte, insbesondere primäre Alkohole mit vorzugsweise 8 bis 22 C-Atomen, insbesondere 8 bis 18 C-Atomen, und durchschnittlich 1 bis 20, vorzugsweise 1 bis 12 Mol Alkylenoxid, besonders bevorzugt 5 bis 15 Mol Alkylenoxid, vorzugsweise Ethylenoxid (EO), pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO oder 5 bis 15 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Als nichtionische Tenside kommen weiterhin auch Substanzen in Betracht, die dem Fachmann gemeinhin auch als nichtionische Emulgatoren bekannt sind. Die nichtionischen Tenside in diesem Sinne enthalten als hydrophile Gruppe z. B. eine Polyolgruppe, eine Polyethergruppe, eine Polyamin- oder eine Polyamidgruppe oder eine Kombination der genannten Gruppen. Solche Verbindungen sind beispielsweise C₈-C₂₂-Alkylmono- und -oligoglycoside und deren ethoxylierte Analoga, Anlagerungsprodukte von 2 bis 30 Mol Ethylenoxid und/oder 0 bis 10, insbesondere 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin sowie Anlagerungsprodukte von 5 bis 60 Mol Ethylenoxid an Rizinusöl und an gehärtetes Rizinusöl.

Auch schwachschäumende nichtionische Tenside können eingesetzt werden, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt. Beispiele hierfür sind Tenside der allgemeinen Formel 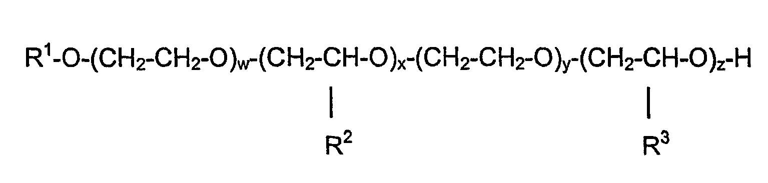 in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃; -CH₂CH₃, -CH₂CH₂-CH₃, CH(CH₃)₂ und die Indizes w, x, y, z unabhängig voneinander für ganze Zahlen von 1 bis 6 stehen. Diese lassen sich durch bekannte Methoden aus den entsprechenden Alkoholen R¹-OH und Ethylen- bzw. Alkylenoxid herstellen. Der Rest R¹ in der vorstehenden Formel kann je nach Herkunft des Alkohols variieren. Werden native Quellen genutzt, weist der Rest R¹ eine gerade Anzahl von Kohlenstoffatomen auf und ist in der Regel unverzeigt, wobei die linearen Resten aus Alkoholen nativen Ursprungs mit 12 bis 18 C-Atomen, z.B. aus Kokos-, Palm-, Talgfett- oder Oleylalkohol, bevorzugt sind. Aus synthetischen Quellen zugängliche Alkohole sind beispielsweise die Guerbetalkohole oder in 2-Stellung methylverzweigte bzw. lineare und methylverzweigte Reste im Gemisch, so wie sie üblicherweise in Oxoalkoholresten vorliegen. Unanbhängig von der Art des zur Herstellung der erfindungsgemäß in den Mitteln enthaltenen nicht-ionischen Tensiden eingesetzten Alkohols sind erfindungsgemäße Mittel bevorzugt, bei denen R¹ in der vorstehenden Formel für einen Alkylrest mit 6 bis 24, vorzugsweise 8 bis 20, besonders bevorzugt 9 bis 15 und insbesondere 9 bis 11 Kohlenstoffatomen steht. Als Alkylenoxideinheit, die alternierend zur Ethylenoxideinheit in den Niotensiden enthalten sein kann, kommt neben Propylenoxid insbesondere Butylenoxid in Betracht. Aber auch weitere Alkylenoxide, bei denen R² bzw. R³ unabhängig voneinander ausgewählt sind aus -CH₂CH₂-CH₃ bzw. CH(CH₃)₂, sind geeignet.

Weiterhin kommen als nichtionische Tenside nichtionische Blockcopolymere in Frage wie sie beispielsweise in WO 00/12660 beschrieben werden, auf die hier vollinhaltlich Bezug genommen wird. Es kann sich hierbei beispielsweise handeln um AB-, AA'B-, ABB'-, ABA'- oder BAB'- Blockcopolymere, wobei A und A' für einen hydrophilen Block und B und B' für einen hydrophoben Block steht. Bei den Blöcken A und A' kann es sich unabhänig voneinander beipielsweise handeln um ein Polyalkylenoxid, insbesondere um ein Polypropylenoxid oder Polyethylenoxid, um Polyvinylpyridin, Polyvinylalkohol, Polymethylvinylether, Polyvinylpyrrolidin oder um ein Polysaccharid. Bei den Blöcken B und B' kann es sich unabhängig voneinander beispielsweise handeln um einen gegebenenfalls substituierten Alkylrest, der beispielsweise erhalten werden kann durch Polymerisation von Einheiten ausgewählt aus der Gruppe bestehend aus 1,3-Butadien, Isopren, alle Konstitumere des Dimethylbutadien, 1,3-Pentadien, 2.4-Hexadien, α-Methylstyrol, Isobutylen, Ethylen, Propylen oder Styrol oder Mischungen davon. Die Molekulargewichte der Blöcke A, A', B und B' betragen vorzugsweise unabhängig voneinander zwischen 500 und 50000 g/mol. Erfindungsgemäß handelt es sich vorzugsweise bei mindestens einem der Blöcke A und A' um ein Alkylenoxid.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Außerdem können als nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein.

Weitere geeignete Tenside sind Polyhydroxyfettsäureamide der nachstehenden Formel, 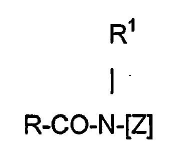 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel, 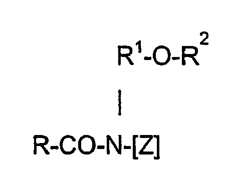 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Weitere einsetzbare nichtionische Tenside sind die endgruppenverschlossenen poly(oxyalkylierten) Tenside der Formel

R¹O[CH₂CH(R³)O]x[CH₂]ₖCH(OH)[CH₂]ⱼOR²

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

In einer besonders bevorzugten Ausführungsform handelt es sich bei den nichtionischen Tensiden um ein Anlagerungsprodukt von Alkylenoxid-, insbesondere Ethylenoxid (EO)- und/oder Propylenoxid (PO)-Einheiten, an Alkylphenole, wobei der Alkylrest des Alkylphenols vorzugsweise zwischen 6 und 18 C-Atome, besonders bevorzugt zwischen 6 und 12 C-Atome, vor allem 8, 9 oder 10 C-Atome umfasst und wobei vorzugsweise zwischen 1 und 18 Ethylenoxid (EO)-Einheiten, besonders bevorzugt zwischen 5 und 15 EO-Einheiten, vor allem 8, 9 oder 10 EO-Einheiten an den Alkylphenol-Rest angelagert sind, wobei die angegebenen Werte Durchschnittswerte sind und wobei der Alkylrest des Alkylphenols linear oder in 2-Stellung methylverzweigt sein kann bzw. lineare und methylverzweigte Reste im Gemisch enthalten kann, so wie sie üblicherweise in Oxoalkoholresten vorliegen. In einer besonders bevorzugten Ausführungsform handelt es sich bei dem nichtionischen Tensid um ein Anlagerungsprodukt von durchschnittlich 9 EO-Einheiten an Nonylphenol, wobei der Alkylrest und der Polyethylenrest vorzugsweise in meta-Stellung zueinander angeordnet sind. Ein solches Produkt ist beispielsweise unter dem Namen Disponil NP9 (Cognis, Deutschland) erhältlich.

In einer weiteren besonders bevorzugten Ausführungsform handelt es sich bei dem nichtionischen Tensid um ein Anlagerungsprodukt von Ethylenoxid (EO)-Einheiten an einen Fettalkohol, wobei der Fettalkohol vorzugsweise zwischen 10 und 22 C-Atome, besonders bevorzugt zwischen 14 und 20 C-Atome, vor allem zwischen 16 und 18 C-Atome umfasst, und wobei vorzugsweise zwischen 4 und 24, besonders bevorzugt zwischen 10 und 22, EO-Einheiten, vor allem 11, 12, 13, 19, 20 oder 21 EO-Einheiten, an den Fettalkohol angelagert sind. Bevorzugte Produkte, die aus einem C₁₆₋₁₈-Alkohol mit 12 bzw. 20 EO-Einheiten bestehen, sind beispielsweise unter den Handelsnamen Eumulgin B1 bzw. Eumulgin B2 (Cognis, Deutschland) erhältlich.

In einer weiteren besonders bevorzugten Ausführungsform handelt es sich bei dem nichtionischen Tensid um ein Anlagerungsprodukt von Ethylenoxid (EO)-Einheiten an einen Fettalkohol, wobei der Fettalkohol vorzugsweise zwischen 8 und 22 C-Atome, besonders bevorzugt zwischen 10 und 20 C-Atome, vor allem zwischen 12 und 18 C-Atome umfasst, und wobei vorzugsweise zwischen 3 und 15, besonders bevorzugt zwischen 5 und 11, EO-Einheiten, vor allem 6, 7, 8, 9 oder 10 EO-Einheiten, an den Fettalkohol angelagert sind. Bevorzugte Produkte, die aus einem C₁₂₋₁₈-Alkohol mit 7 bzw. 9 EO-Einheiten bestehen, sind beispielsweise unter den Handelsnamen Dehydol LT7 und Dehydol 100 (Cognis, Deutschland) erhältlich.

In einer weiteren besonders bevorzugten Ausführungsform handelt es sich bei dem nichtionischen Tensid um ein Anlagerungsprodukt von Ethylenoxid (EO)-Einheiten an einen Fettalkohol, wobei der Fettalkohol vorzugsweise zwischen 18 und 26 C-Atome, besonders bevorzugt zwischen 20 und 24 C-Atome, vor allem 22 C-Atome umfasst, und wobei vorzugsweise zwischen 6 und 16, besonders bevorzugt zwischen 8 und 12, EO-Einheiten, vor allem 9, 10 oder 11 EO-Einheiten, an den Fettalkohol angelagert sind. Ein bevorzugtes Produkt, das aus einem C₂₂-Alkohol mit 10 EO-Einheiten besteht, ist beispielsweise unter den Handelsnamen Mergital B10 (Cognis, Deutschland) erhältlich.

In einer weiteren besonders bevorzugten Ausführungsform handelt es sich bei dem nichtionischen Tensid um ein Anlagerungsprodukt von Ethylenoxid (EO)- und Propylenoxid (PO)-Einheiten an einen Fettalkohol, wobei der Fettalkohol vorzugsweise zwischen 6 und 18 C-Atome, besonders bevorzugt zwischen 10 und 16 C-Atome, vor allem zwischen 10 und 12 oder zwischen 12 und 14 C-Atome umfasst, und wobei vorzugsweise zwischen 1 und 10, besonders bevorzugt zwischen 3 und 7, vor allem 4, 5 oder 6, EO-Einheiten sowie vorzugsweise zwischen 1 und 10, besonders bevorzugt zwischen 2 und 6, vor allem 3, 4, 5 oder 6, PO-Einheiten an den Fettalkohol angelagert sind. In einer bevorzugten Ausführungsform handelt es sich hierbei bei dem nichtionischen Tensid um ein Block-Copolymer, bei dem vorzugsweise die EO-Einheiten an den Fettalkohol angelagert sind und die PO-Einheiten auf die EO-Einheiten folgen und wobei der Alkylrest des Fettalkohols linear oder in 2-Stellung methylverzweigt sein kann bzw. lineare und methylverzweigte Reste im Gemisch enthalten kann, so wie sie üblicherweise in Oxoalkoholresten vorliegen. Ein bevorzugtes Produkt, das aus einem C₁₂-C₁₄-Alkohol mit 5 EO- und 4 PO-Einheiten besteht, ist beispielsweise unter dem Namen Dehyppon LS 54 (Cognis, Deutschland) erhältlich. Ein weiteres bevorzugtes Produkt, das aus einem C₁₀₋₁₂-Alkohol mit 5 EO- und 5 PO-Einheiten besteht, ist beispielsweise unter dem Namen Biodac 2/32 (Cognis, Deutschland) erhältlich.

In einer weiteren erfindungsgemäß bevorzugten Ausführungsform handelt es sich bei dem nichtionischen Tensid um ein fluoriertes bzw. fluor-haltiges nichtionisches Tensid. Besonders bevorzugt handelt es sich hierbei um ein Anlagerungsprodukt von Alkylenoxid-Einheiten, insbesondere Ethylenoxid (EO)- und/oder Propylenoxid (PO)-Einheiten an einen Alkylalkohol, wobei der Alkylalkohol vorzugsweise zwischen 4 und 20 C-Atome, besonders bevorzugt zwischen 6 und 18 C-Atome umfasst, und wobei vorzugsweise zwischen 1 und 18, besonders bevorzugt zwischen 2 und 16 EO-Einheiten an den Alkylalkohol angelagert sind und wobei die Verbindung, vorzugsweise der Alkylrest, mindestens ein Fluor-Atom, vorzugsweise mindestens 5 Fluor-Atome, insbesondere zwischen 5 und 30 Fluor-Atome, enthält. In einer besonders bevorzugten Ausführungsform handelt es sich bei der Verbindung bzw. Mischung von Verbindungen um eine mit der Formel F(CF₂F₂)₁₋₇CH₂CH₂O(CH₂CH₂O)₁₋₁₅H. Ein solches nichtionisches Tensid ist beispielsweise unter dem Namen Zonyl FSO 100 (Dupont, Frankreich) erhältlich.

Die Hydroxy-Gruppen der zuvor genannten Hydroxy-Gruppen tragenden nichtionischen Tenside können erfindungsgemäß in einer besonderen Ausführungsform auch teilweise oder vollständig verethert oder verestert sein. Es kann hierbei insbesondere eine Etherbindung zu einer C₁₋₆-Alkylgruppe, vorzugsweise zu einer Methyl-, Ethyl-, iso-Propyl- oder tert-Butyl-Gruppe, vorliegen. Unter den Esterbindungen sind solche zu einer C₁₋₆-Alkancarbonsäure, insbesondere zu Essigsäure oder Maleinsäure, bevorzugt.

In einer bevorzugten Ausführungsform werden die nichtionischen Tenside in Träger-gebundener Form eingesetzt. Als Träger kommen hierbei insbesondere Moleküle in Frage, die eine kovalente Bindung und/oder eine interkalierende Bindung der nichtionischen Tenside ermöglichen. Als Beispiele für die erste Art von Trägem seien makromolekulare Moleküle mit Säurefunktion genannt, die die Bindung von Hydroxy-Gruppen haltigen nichtionischen Tensiden in Form von Ester-Bindungen ermöglichen. Als Beispiele für die zweite Art von Trägern seien sogenannte Käfigmoleküle genannt, die die Aufnahme der nichtionischen Tenside in die Käfigstruktur ermöglichen.

Als Ester der nichtionischen Tenside werden vorzugsweise solche mit Kieselsäuren gemäß den Formeln I und II eingesetzt. Die Herstellung der Kieselsäureester gelingt insbesondere durch einfache Umesterung von Kieselsäureestern (n=1) bzw. Oligokieselsäureestern (n>1) niederer Alkohole mit den nichtionischen Tensiden. Je nach Reaktionszeit und -bedingungen werden die niederen Alkohole abgespalten und die gewünschten Wirkstoffe gebunden, wobei die Alkohole entlang der Si-O-Si-Kette leichter ausgetauscht werden als die terminalen Alkohole.

Bevorzugt werden Kieselsäureester gemäß einer der Formeln (I) oder (II) und/oder deren Mischungen eingesetzt. 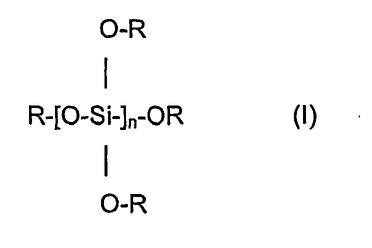 und 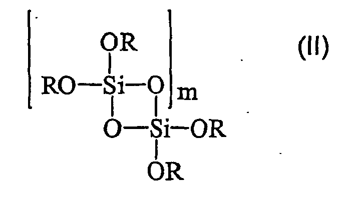 wobei mindestens ein R ein nichtionisches Tensid ist und alle anderen R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste, Terpenalkohole, sowie Polymere enthält, und m Werte aus dem Bereich 1 bis 20 und n Werte aus dem Bereich 1 bis 100 annimmt.

Nach einer weiteren bevorzugten Ausführungsform sind mindestens zwei oder drei Reste R ein nichtionisches Tensid.

Die Oligomerisierungsgrade "n" der erfindungsgemäßen Kieselsäureester liegen vorzugsweise zwischen 1 und 20. In besonders bevorzugten Verbindungen nimmt n Werte zwischen 1 und 15, insbesondere zwischen 1 und 12 und vor allem zwischen 1 und 10, insbesondere die Werte 4, 5, 6, 7 oder 8, an.

Die erfindungsgemäß verwendeten Kieselsäureester zeichnen sich durch eine gute Hydrolysestabilität aus und können auch in wäßrigen Medien bzw. in Herstellprozessen für Granulate, Dichtungsmassen, etc. eingesetzt werden, ohne dabei übermäßige Aktivitätsverluste zu erleiden. Die Freisetzung des Wirkstoffs aus den erfindungsgemäßen Stoffen erfolgt dadurch langsam und in vergleichsweise geringen Mengen, so dass über einen längeren Zeitraum kontinuierlich eine allmähliche Freisetzung der Wirkstoffe aus den Produkten erfolgt.

Nach einer besonders bevorzugten Ausführungsform können sich ein oder mehrere Polymerreste an den Kieselsäureestern befinden. Bevorzugt werden solche Polymere zur Herstellung der Kieselsäureester eingesetzt, die freie Hydroxylgruppen enthalten. Insbesondere sind der oder die Polymerrest(e) ausgewählt aus Stärke und/oder dessen Derivaten, Cellulose und/oder dessen Derivaten, Polyvinylalkohol, Polyolen, Hydroxypolydimethylsiloxanen (ganz besonders α,ω-Dihydroxypolydimethylsiloxane) und Polyphenolen, insbesondere Polyvinylalkohol. Insbesondere ist es bevorzugt, wenn sich ein Polymerrest an den nichtionische Tenside tragenden Kieselsäureestern befindet. Für die Verwendung in Dichtungsmassen ist es besonders bevorzugt eher kurzkettige Polymere einzusetzen.

Diese spezielle Ausführungsform hat den Vorteil, dass man die Kieselsäureester je nach Anwendungsgebiet individuell auf den Anwendungszweck bzw. die - gegebenheiten anpassen kann. Beispielsweise sind solche Polymere besonders geeignet, die Einarbeitbarkeit der Stoffe zu verbessern, die Haftung, insbesondere an Oberflächen, zu erhöhen und die Freisetzungseigenschaften gewünschtermaßen zu beeinflussen.

Des weiteren können auch Ester der nichtionischen Tenside mit Polymeren eingesetzt werden. Auch für diese Stoffe ergeben sich bessere Anpassbarkeit an den Anwendungszweck, beispielsweise ein besseres Aufziehen oder Anhaften an Oberflächen oder günstigere Bedingungen für die Einarbeitbarkeit. Die Hydrolyse dieser Esterbindung, z. B. bei sich wiederholendem Kontakt mit Wasser, setzt langsam die nichtionischen Tenside frei, die dann ihre anti-adhäsive Wirkung entfalten können.

Besonders bevorzugt werden solche Stoffe durch Reaktion der nichtionischen Tenside mit solchen Polymeren durchgeführt, die funktionelle Gruppen tragen, die insbesondere ausgewählt sind aus Säuregruppen, Säurechloridgruppen, Estergruppen, primären, sekundären und tertiären Amidgruppen.

Bevorzugterweise werden als Polymere erfindungsgemäß Polyacrylsäure, Polyacrylsäureester, Polymethacrylsäure, Polymethacrylsäureester, Polycarbonsäuren, (insbesondere Carboxymethylcellulose) sowie Copolymere aus dem zugrundeliegenden Monomer (auch mit anderen als den genannten Monomeren) und primäre, sekundäre oder tertiäre Polyacrylamide eingesetzt. Insbesondere sind dabei Kettenlängen von ca. 2000 bis 300000 g/mol bevorzugt.

Gemäß einer weiteren bevorzugten Ausführungsform wird der Polymer-Ester hergestellt durch Umsetzung des Wirkstoffs mit Monomeren oder Polymeren, die ein oder mehrere Isocyanatgruppen tragen. Die sich durch die Reaktion von einer Alkoholfunktion mit einer Isocyanatgruppe ergebenen Urethane hydrolysieren ebenfalls langsam und geben den Wirkstoff kontrolliert frei.

Bevorzugt ist der Einsatz von monomeren aliphatischen oder aromatischen Mono-, Di- und/oder Triisocyanaten. Die daraus entstehenden Urethane bzw. Polyurethane (bei Verwendung von Isocyanaten mit mehreren Isocyanatgruppen) können ebenfalls hydrolysieren und die Wirkstoffe langsam freisetzen.

Als Monoisocyanate sind beispielsweie die linearen oder verzweigten aliphatischen Monoisocyanate mit 6 bis 44 C-Atomen, beispielsweise Hexylisocyanat, Heptylisocyanat, Octylisocyanat, Nonylisocyanat, Decylisocyanat, Undecylisocyanat, Dodecylisocyanat, Tridecylisocyanat, Quaterdecylisocyanat, Pentadecylisocyanat, Hexadecylisocyanat, Heptadecylisocyanat, Octadecylisocyanat und die entsprechenden höheren Homologe dieser Reihe bevorzugt. Ebenfalls bevorzugt sind aromatische Monoisocyanate wie Phenylisocyanat, Benzylisocyanat oder Biphenylisocyanat.

Als Diisocyanate (Q(NCO)₂) sind insbesondere solche bevorzugt, bei denen Q ausgewählt ist aus einem aliphatischen, ggf. substituierten Kohlenwasserstoffrest mit 4 bis etwa 15 Kohlenstoffatomen, einem aromatischen, ggf. substituierten Kohlenwasserstoffrest mit 6 bis etwa 15 Kohlenstoffatomen oder einen ggf. substituierten araliphatischen Kohlenwasserstoffrest mit 7 bis etwa 15 Kohlenstoffatomen. Beispielsweise genannt seien hier Tetramethylendiisocyanat, Hexamethylendiisocyanat, Dodecamethylendiisocyanat, Dimerfettsäurediisocyanat, 1,4-Diisocyanato-cyclohexan, 1-Isocyanato-3,3,5-trimethyl-5-isocyanato-methylcyclohexan (IDPI), 4,4'-Diisocyanato-dicyclohexylmethyl, 4,4'-Diisocyanatodicyclohexylpropan-2,2, 1,3- und 1,4-Diisocyanatobenzol, 2,4- oder 2,6-Diisocyanatotoluol oder deren Gemische, 2,2'-, 2,4 oder 4,4'-Diisocyanato-diphenylmethan, Tetramethylxylylendiisocyanat, p-Xylylendiisocyanat sowie aus diesen Verbindungen bestehende Gemische.

Besonders bevorzugt sind Toluendiisocyanat, Hexamethylendiisocyanat und meta-Tetramethylxylylendiisocyanat.

Als Triisocyanate kommen dabei hauptsächlich aromatische Triisocyanate in Frage wie zum Beispiel der Thiophosphorsäure-tris-(p-Isocyanato-Phenylester), das Triphenylmethan-4,4',4"-Triisocyanat sowie insbesondere die verschiedenen isomeren trifunktionellen Homologen des Diphenylmethandiisocyanats (MDI).

Weiterhin sind als Triisocyanate auch Addukte aus Diisocyanaten und niedermolekularen Triolen geeignet, insbesondere die Addukte aus aromatischen Diisocyanten und Triolen wie zum Beispiel Trimethylolpropan oder Glycerin. Auch bei diesen Addukten gelten die oben genannten Einschränkungen bezüglich des Diisocyanatgehaltes sowie des Gehaltes an Polyisocyanaten mit einer Funktionalität >3.

Auch aliphatische Triisocyanate wie zum Beispiel das Biuretisierungsprodukt des Hexamethylendiisocyanates (HDI) oder das Isocyanuratisierungsprodukt des HDI oder auch die gleichen Trimerisierungsprodukte des Isophorondiisocyanats (IPDI) sind für die erfindungsgemäßen Zusammensetzungen geeignet.

Polyisocyanate sind die Dimerisierungs- oder Trimerisierungsprodukte der bereits als bevorzugt genannten Diisocyanate. Beispiele für geeignete Isocyanate sind die Dimerisierungs- oder Trimerisierungsprodukte der Diisocyanate 2,4-Toluylendiisocyanat (2,4-TDI), 2,6-Toluylendiisocyanat (2,6-TDI), oder ein Gemisch der genannten Isomeren, 2,2'-Diphenylmethandiisocyanat (2,2'-MDI), 2,4'-Diphenylmethandiisocyanat (2,4'-MDI), 4,4'-Diphenylmethandiisocyanat (4,4'-MDI), 1,5-Naphthylendiisocyanat (NDI), 1,4-Phenylendiisocyanat, 1,3-Tetramethylxylylendiisocyanat (TMXDI), hydriertes MDI (HMDI), Isophorondiisocyanat (IPDI), Hexamethylendiisocyanat-1,6 (HDI), 2-Isocyanatopropylcyclohexylisocyanat (IPCI), 2-Butyl-2-ethyl-pentamethylendiisocyanat (BEPDI), Lysindiisocyanat (LDI), 1,12-Dodecyldiisocyanat, Cyclohexyl-1,3- oder -1,4-diisocyanat, 2-Methylpentamethylendiisocyanat (MPDI) oder dergleichen, beispielsweise enthaltend Urethan-, Allophanat-, Harnstoff-, Biuret-, Uretidon-, Carbidiimid- oder Ketonimingruppen wie sie durch Dimerisierung oder Trimerisierung der oben genannten Diisocyanate entstehen. Besonders geeignet sind oligomere oder polymere Isocyanatgruppen tragende Verbindungen, wie sie beispielsweise bei der Isocyanatherstellung anfallen oder als Restprodukte bei der Destillierung von Isocyanatrohprodukten im Destillationssumpf verbleiben. Beispiele für in diesem Zusammenhang besonders geeignete Materialien sind Roh-MDI, wie es direkt nach der Herstellung von MDI erhältlich ist, und Polymer-MDI, wie es nach der Destillation von MDI aus dem Roh-MDI im Destillationssumpf verbleibt.

Es ist bevorzugt, eine entsprechende Menge an nichtionischen Tensid zu den Monomeren hinzuzugeben und so entsprechende Monomere zu erzeugen. So können Stoffe erzeugt werden, die je nach verwendeten Monomeren (Monoisocyanate, Diisocyanate, Triisocyanate oder Polyisocyanate) ein oder mehrere, insbesondere ein, zwei oder drei freisetzbare Wirkstoffe tragen. Es ist dabei auch möglich, über eine Polymerisationsreaktion eine Polymerkette mit endständigen Wirkstoffresten zu erzeugen.

Solche Monomere bzw. Polymere können beispielsweise in Dichtungsmassen direkt in der Kartusche oder in einem separaten Kompartiment als Additive eingesetzt werden. Ebenfalls können auch bei der Produktion der Dichtungsmassen, insbesondere bei solchen auf Urethanbasis, die entsprechenden nichtionischen Tenside direkt zu den Monomeren der Dichtungsmassen zugegeben werden. Der Einsatz der Reaktionsprodukte aus Mono-, Di- und/oder Triisocyanate mit nichtionischen Tensiden oder dessen Derivaten in Dichtungsmassen ist besonders bevorzugt.

Als Kettenverlängerungsmittel, die im Rahmen einer Polymerisationsreaktion zur Herstellung der erfindungsgemäß zu verwendenden Stoffe zusätzlich eingesetzt werden können, eignen sich beispielsweise mehrwertige Alkohole wie Ethylenglykol, Propylenglykol, Propandiol-1,3, Butandiol-1,4, Hexandiol-1,6, Trimethylolpropan, Glyzerin, Pentaerythrit, Sorbit, Mannit oder Glucose. Auch niedermolekulare Polyesterdiole wie Bernsteinsäure-, Glutarsäure- oder Adipinsäure-bis-(hydroxyethyl)-ester, oder ein Gemisch aus zwei oder mehr davon, oder niedermolekulare, Ethergruppen aufweisende Diole, wie Diethylenglykol, Triethylenglykol, Tetraethylenglykol, Dipropylenglykol, Tripropylenglykol oder Tetrapropylenglykol können mit verwendet werden. Ebenfalls geeignet sind Amine wie Ethylendiamin, Hexamethylendiamin, Piperazin, 2,5-Dimethylpiperazin, 1-Amino-3-aminomethyl-3,5,5-trimethylcyclohexan (Isophorondiamin, IPDA), 4,4'-Diamino-dicyclohexylmethan, 1,4-Diaminocyclohexan, 1,2-Diaminopropan, Hydrazin, Hydrazinhydrat, Aminosäurehydrazide wie 2-Aminoessigsäurehydrazid oder Bis-hydrazide wie Bernsteinsäurebishydrazid. Die Mitverwendung von im Sinne einer Isocyanat-Polyadditionsreaktion tri- oder höherfunktionellen Verbindungen in geringen Anteilen ist zur Erzielung eines gewissen Verzweigungsgrades ebenso möglich wie die bereits erwähnte mögliche Mitverwendung von tri- oder höherfunktionellen Polyisocyanaten zum gleichen Zweck. Einwertige Alkohole wie n-Butanol oder n-Dodecanol und Stearylalkohol können in geringen Mengen mitverwendet werden.

Unter Käfigmolekülen sind im erfindungsgemäßen Zusammenhang insbesondere solche organischen makrocyclischen Moleküle zu verstehen, die eine käfigartige, räumliche Struktur aufweisen und in der Lage sind, als sogenannte Wirtsmoleküle ein oder mehrere sogenannte Gastmoleküle einzuschließen. Bevorzugt wird jeweils nur ein Gastmolekül eingeschlossen.

Die gezielte, langsame Freisetzung der nichtionischen Tenside kann hierbei über Gleichgewichtseinstellung aus einer (häufig nicht kovalenten) Bindung oder durch Komplexierung der Verbindung aus einem Käfigmolekül stattfinden.

Die Einarbeitbarkeit der beladenen Käfigmoleküle in die erfindungsgemäßen Produkte, insbesondere in solche mit hydrophoberem Charakter, ist aufgrund der eher hydrophoben Außenhülle der Käfigsubstanzen besonders gut.

Ein besonders großer Vorteil der Verwendung von Käfigmolekülen ist, dass es möglich ist, die herausdiffundierten Substanzen nach einer längeren Zeit in den Produkten wieder zu ersetzen, indem die Käfigmoleküle wieder aufgeladen werden. Insbesondere sind hierzu konzentrierte Lösungen der Wirkstoffe geeignet. Es ist in diesem Sinne ebenfalls möglich, Produkte herzustellen, die nicht von vornherein die freien Wirkstoffe, also die nichtionischen Tenside, komplexiert bzw. gebunden in den Käfigmolekülen enthalten, sondern erst in der Anwendungssituation durch diese beladen werden. Formulierungstechnisch ist das für dem Fachmann bekannte Einsatzgebiete sinnvoll.

Als organische Käfigmoleküle seien Cucurbiturile, Calixarene, Calixresorcarene, Cyclodextrine, Cyclophane, Kronenether, Fullerene, Cryptophane, Carceranden, Hemicarceranden, Cyclotriveratrylene, Spheranden und Cryptanden genannt.

Besonders bevorzugt sind erfindungsgemäß die Cucurbiturile, Calixarene und Calixresorcarene, ganz besonders Cucurbiturile.

Cucurbiturile und ihre Herstellung sind in der Literatur beschrieben, beispielsweise in der WO 00/68232 und der EP-A 1 094 065 sowie der dort zitierten weiteren Literatur. Unter einem im Sinne der Erfindung einsetzbaren Cucurbituril ist grundsätzlich jeder Stoff zu verstehen, der in der Literatur als zu dieser Verbindungsklasse gehörig beschrieben ist. Definitionsgemäß einbezogen sind hier die in der WO 00/68232 beschriebenen Cucurbiturile und substituierten Cucurbiturile sowie die in der EP-A 1 094 065 beschriebenen Cucurbituril-Derivate. Anstelle eines einheitlichen Cucurbiturils, substituierten Cucurbiturils oder Cucurbituril-Derivats können ebenso Mischungen von zwei oder mehr derartiger Verbindungen eingesetzt werden. Soweit im folgenden Text von einem Cucurbituril die Rede ist, und nicht ausdrücklich etwas anderes angegeben ist, ist darunter in gleicher Weise ein chemisch einheitliches Cucurbituril oder auch ein Gemisch zweier oder mehrerer Cucurbiturile, substituierter Cucurbiturile und/oder Cucurbituril-Derivate zu verstehen. Dementsprechend beziehen sich Mengenangaben von Cucurbiturilen, soweit nicht ausdrücklich etwas anderes angegeben ist, stets auf die Gesamtmenge des einen oder der mehreren Cucurbiturile, substituierten Cucurbiturile und/oder Cucurbituril-Derivate.

Im Sinne der vorliegenden Erfindung bevorzugt sind Cucurbit[n]urile der Ringgröße 5 bis 11, sowie deren Gemische, wobei Cucurbit[6]uril sowie Mischungen mit einem überwiegenden Anteil an Cucurbit[6]uril besonders bevorzugt sind.

Weiterhin können Calix[n]arene gemäß Formel (VIII) eingesetzt werden. 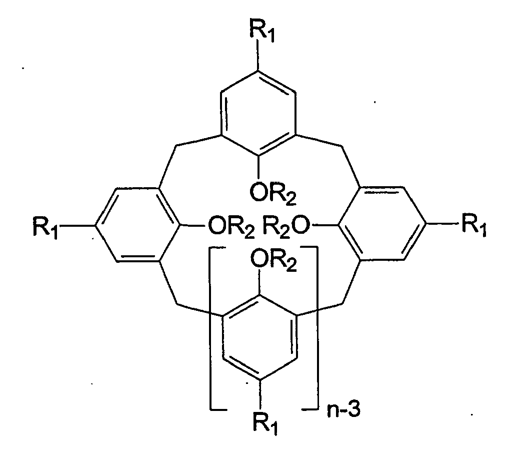 wobei
R₁ ausgewählt ist aus R₁ = H, Alkyl, Aryl, Alkenyl, Alkinyl sowie substituierten Alkylen, Arylen, Alkenylen, Alkinylen, die Gruppen tragen, die ausgewählt sind aus -OH, -OR', - NH₂, -NHR', -NR'R", NR'R"R"'⁺ ,NO₂, Halogen, SO₃H, SO₃M (M = Alkalimetalle, Erdalkalimetalle), Carbonsäuren, Ketonen, Aldehyden, Amiden, Estern, -SO₂NH₂, - SO₂NHR, -SO₂NR'R", -SO₂Halogen, schwefel-, phosphor-, siliziumhaltigen Gruppen.
   und
R₂ ausgewählt ist aus R₂ = H, Alkyl, Aryl, Alkenyl, Alkinyl, sowie substituierten Alkylen, Arylen, Alkenylen, Alkinylen , die Gruppen tragen, die ausgewählt sind aus -OH, -OR', - NH₂, -NHR', -NR'R", -NR'R"R"'⁺, -NO₂, Halogen, -SO₃H, -SO₃M (M = Alkalimetalle, Erdalkalimetalle), Carbonsäuren, Ketonen, Aldehyden, Amiden, Estern, -SO₂NH₂, - SO₂NHR', -SO₂NR'R", -SO₂Halogen, schwefel-, phosphor-, oder siliziumhaltigen Gruppen,
   wobei R', R", R'" unabhängig von einander ausgewählt sind aus H, Alkyl, Aryl, Alkenyl, Alkinyl, substituierten Alkyle, Aryle, Alkenyle, Alkinyle

Bevorzugt sind dabei Calixarene gemäß Formel (VIII) für die gilt:
R₁ ausgewählt ist aus R₁ = H, Alkyl, Aryl, Alkenyl, Alkinyl sowie substituierten Alkylen, Arylen, Alkenylen, Alkinylen, die Gruppen tragen, die ausgewählt sind aus -OH, -OR', - NH₂, -NHR', -NR'R", NR'R"R"'⁺, NO₂, Halogen, SO₃H, Carbonsäuren, Ketonen, Aldehyden, Amiden, Estern, -SO₂NR'R".
   und
R₂ ausgewählt ist aus R₂ = H, Alkyl, Aryl, Alkenyl, Alkinyl, sowie substituierten Alkylen, Arylen, Alkenylen, Alkinylen , die Gruppen tragen, die ausgewählt sind aus -OH, -OR', - NH₂, -NR'R", -NR'R"R"'⁺, -NO₂, Halogen, -SO₃H, Carbonsäuren, Ketonen, Aldehyden, Amiden, Estern, -SO₂NR'R".
   wobei R', R", R'" unabhängig von einander ausgewählt sind aus H, Alkyl, Aryl, Alkenyl, Alkinyl sowie substituierten Alkylen, Arylen, Alkenylen, Alkinylen.

Im Sinne der vorliegenden Erfindung bevorzugt sind Calix[n]arene der Ringgröße n= 4 bis 12, sowie deren Gemische, wobei Calix[6]- und/oder Calix[4]arene sowie Mischungen mit einem überwiegenden Anteil an Calix[6]- und/oder Calix[4]arene besonders bevorzugt sind.

Darüber hinaus sind Calix[n]resorcarene, auch als Resorcinarene bekannt, gemäß Formel IX zu verwenden. N gibt dabei die Zahl der Kettenglieder an und kann 4 oder 6 betragen. 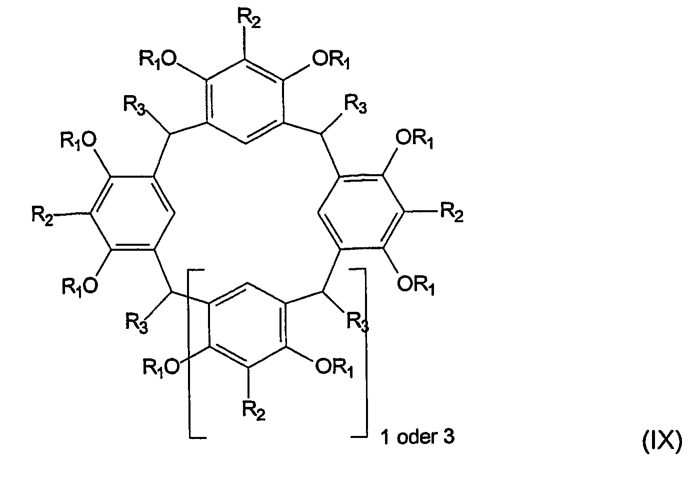 wobei R₁, R₂ und R₃ ausgewählt sind aus:
R₁ = H, Alkyl, Aryl, Alkenyl, Alkinyl, sowie substituierten Alkylen, Arylen, Alkenylen, Alkinylen, die Gruppen tragen, die ausgewählt sind aus -OH, -OR, -NH₂, -NHR', - NR'R", NR'R"R"'⁺, -NO₂, Halogen, SO₃H, SO₃M (M = Alkalimetalle, Erdalkalimetalle), Carbonsäuren, Ketone, Aldehyde, Amide, Ester, SO₂NH₂, SO₂NHR, SO₂NR₂, SO₂Halogen, schwefel-, phosphor-, siliziumhaltige Gruppen
   und
R₂,R₃ unabhängig von einander ausgewählt sind aus R₂, R₃= H, Alkyl, Aryl, Alkenyl, Alkinyl, sowie substituierten Alkylen, Arylen, Alkenylen, Alkinylen, die Gruppen tragen, die ausgewählt sind aus -OH, -OR, -NH₂, -NHR, NR'R", NR'R"R"'⁺, -NO₂, Halogen, - SO₃H, -SO₃M (M = Alkalimetalle, Erdalkalimetalle), Carbonsäuren, Ketonen, Aldehyden, Amiden, Estern, -SO₂NH₂, -SO₂NHR, -SO₂NR₂, -SO₂Halogen, schwefel-, phosphor-, siliziumhaltige Gruppen,
   und wobei R', R", R'" unabhängig von einander ausgewählt sind aus H, Alkyl, Aryl, Alkenyl, Alkinyl sowie substituierten Alkylen, Arylen, Alkenylen, Alkinylen.

Bevorzugt sind Calix[4]resorcarene und/oder Calix[6]resorcarene gemäß Formel (IX), für die gilt, dass
R₁ ausgewählt ist aus R₁ = H, Alkyl, Aryl, Alkenyl, Alkinyl sowie substituierten Alkylen, Arylen, Alkenylen, Alkinylen, die Gruppen tragen, die ausgewählt sind aus -OH, -OR', - NH₂, -NHR', -NR'R", NR'R"R"'⁺, NO₂, Halogen, SO₃H, Carbonsäuren, Ketonen, Aldehyden, Amiden, Estern, -SO₂NR'R".
   und
R₂,R₃ unabhängig von einander ausgewählt sind aus R₂, R₃= H, Alkyl, Aryl, Alkenyl, Alkinyl, sowie substituierten Alkylen, Arylen, Alkenylen, Alkinylen , die Gruppen tragen, die ausgewählt sind aus -OH, -OR', -NH₂, -NR'R", -NR'R"R"'⁺, -NO₂, Halogen, - SO₃H, Carbonsäuren, Ketonen, Aldehyden, Amiden, Estern, -SO₂NR'R".
   wobei R', R", R"' unabhängig von einander ausgewählt sind aus H, Alkyl, Aryl, Alkenyl, Alkinyl sowie substituierten Alkylen, Arylen, Alkenylen, Alkinylen.

Insbesondere ist es bevorzugt, wenn R₂ = R₃ ist, d.h. R₂ und R₃ die gleichen Substitutenten darstellen.

Die nichtionischen Tenside und/oder ihre Träger-gebundenen Formen werden erfindungsgemäß vorzugsweise in Mengen bis zu 20 Gew.-%, besonders bevorzugt in Mengen zwischen 0,001 und 3 Gew.-%, vor allem in Mengen zwischen 0,01 und 1,5 Gew.-% eingesetzt.

Die Mengen, die im Endprodukt zum gewünschten Ergebnis führen, können bedeutend geringer als die angegebenen sein, da für viele Produkte Verdünnungen berücksichtigt werden müssen. Für Waschmittel muss beispielsweise mit einem Verdünnungsfaktor (Verhältnis Waschmittelkonzentrat : Wasser) von 1:20 bis zu 1:200 gerechnet werden. Häufig liegt das Verdünnungsverhältnis für Waschmittel zwischen 1:60 und 1:100, beispielsweise 1:80.

### Mikroorganismen

In einer bevorzugten Ausführungsform sind unter Mikroorganismen Bakterien und Pilze zu verstehen. Besonders bevorzugte Pilze sind hierbei Hefen, Schimmelpilze, Dermatophyten und keratinophile Pilze.

Gemäß einer besonders bevorzugten Ausführungsform wird durch die Verwendung von nichtionischen Tensiden die Anhaftung von Bakterien vermindert, insbesondere die Anhaftung von gramnegativen und grampositiven Bakterien, vor allem die Anhaftung von pathogenen Bakterien ausgewählt aus Propionibacterium acnes, Stapylococcus aureus, Streptokokken Gruppe A (beta-hämolysierende S.), S. pyogenes, Corynebacterium spp. (insbesondere C. tenuis, C. diphtheriae, C. minutissimum), Micrococcus spp. (insbesondere M. sedentarius), Bacillus anthracis, Neisseria meningitidis, N. gonorrhoeae, Pseudomonas aeruginosa, P. pseudomallei, Borrelia burgdorferi, Treponema pallidum, Mycobacterium tuberculosis, Mycobacterium spp., Escherichia coli sowie Streptococcus spec. (insbesondere S. gordonii, S. mutans), Actinomyces spec. (insbesondere A. naeslundii), Salmonella spec., Actinobacteria (insbesondere Brachybacterium spec.), alpha-Proteobacteria (insbesondere Agrobacterium spec.), beta-Proteobacteria (insbesondere Nitrosomonas sec.), Aquabacterium spec., Hydrogenophaga, gamma-Proteobacteria, Stenotrophomonas spec., Xanthomonas spec. (campestris), Neisseria spec., Haemophilus spec. sowie alle Mikroorganismen, die von Paster et al. (J. Bac. 183 (2001) 12, 3770-3783) beschrieben werden.

Hefen im erfindungsgemäßen Zusammenhang sind einzellige Pilze, die sich überwiegend durch Sprossung vermehren. Hefepilze stellen keine eigenständige taxonomische Kategorie im System der Pilze dar. Systematisch werden die meisten Hefen den Endomycetes zugeordnet. Daneben treten aber auch bei verschiedenen anderen Pilzen im Entwicklungszyklus oder unter bestimmten Umweltbedingungen Sprosszellstadien auf, die als Hefestadien bezeichnet werden. Solche einzellig, hefeartig sprossende Wuchsformen treten bei den Ascomyceten, aber auch bei den Zygomyceten, Basidomyceten und Deuteromyceten auf. Erfindungsgemäß sind auch alle diese Wuchsformen unter Hefen zu verstehen.

Nach einer weiteren besonderen Ausführungsform wird durch die Verwendung von nichtionischen Tensiden die Anhaftung von humanpathogenen Pilzen vermindert. Dazu sind zum Beispiel die humanpathogenen Spezies der Pilze aus den Klassen Ascomycota, Basidomycota, Deuteromycota und Zygomycota zu zählen, insbesondere die humanpathogenen Formen von Candida.

Die humanpathogenen Candida Spezies besiedeln auch bei gesunden Menschen Haut und Schleimhäute. Bei starker Vermehrung der Pilzzellen, z.B. nach Schädigung der bakteriellen Schleimhautflora durch Antibiotika, verursachen sie aber lokale Entzündungen, die auch als Soor bezeichnet werden. Insbesondere treten diese im Mund- und Genitalbereich auf (sogenannter Mund- bzw. Vaginal-Soor). Bekannt sind auch Haut- und Windelsoor. Die Schleimhaut ist gerötet, es zeigen sich Läsionen, ein weißer Belag und Juckreiz treten auf.

Insbesondere wird erfindungsgemäß die Anhaftung von Pilzen der Spezies Candida (im weiteren mit C. abgekürzt) vermindert, die ausgewählt aus C. aaseri, C. actiscondensi, C. acutus, C. agrestis, C. albicans, C. amapae, C. anatomiae, C. ancudensis, C. antarctica, C. antillancae, C. apicola, C. apis, C. aquaetextoris, C. aquatica, C. atlantica, C. atmosphaerica, C. auringiensis, C. azyma, C. beechii, C. benhamii, C. bertae, C. berthetii, C. blankii, C. boidinii, C. boleticola, C. bombi, C. bondarzewiae, C. brumptii, C. buffonii, C. buinensis, C. cacaoi, C. cantarellii, C. capsuligena, C. cariosilignicola, C. caseinolytica, C. castellii, C. catenulata, C. chalmersi, C. chilensis, C. chiropterorum, C. ciferii, C. claussenii, C. coipomensis, C. colliculosa, C. conglobata, C. curiosa, C. cylindracea, C. dendrica, C. dendronema, C. deserticola, C. diddensiae, C. diffluens, C. diversa, C. drymisii, C. dubliniensis, C. edax, C. entomophila, C. eremophila, C. ergatensis, C. ernobii, C. etchellsii, C. etchellsii, C. ethanolica, C. ethanothermophilum, C. evantina, C. fabianii, C. famata, C. fennica, C. flareri, C. fluviotilis, C. fragariorum, C. fragi, C. fragicola, C. freyschussii, C. friedrichii, C. fructus, C. fusiformata, C. geochares, C. glabrata, C. glaebosa, C. graminis, C. gropengiesseri, C. guilliermondii, C. haemulonii, C. hellenica, C. heveanensis, C. holmii, C. homilentoma, C. humicola, C. humilis, C. iberica, C. incommunis, C. inconspicua, C. ingens, C. insectalens, C. insectamans, C. insectorum, C. intermedia, C ishiwadae, C. japonica, C. javanica, C. karawaiewii, C. kefyr, C. kruisii, C. krusei, C. krusoides, C. lactiscondensi, C. lambica, C. laureliae, C. lipolytica, C Ilanquihuensis, C. lodderae, C. lusitaniae, C. magnoliae, C. malicola, C. maltosa, C. maris, C. maritima, C. melibiosica, C. melinii, C. membranaefaciens, C. mesenterica, C. methanosorbosa, C milleri, C. mogii, C. molischiana, C. monosa, C. montana, C. mucilaginosa, C. multis-gemmis, C. musae, C. muscorum, C. mycoderma, C. naeodendra, C. nakasei, C. nemodendra, C. nitratophila, C. norvegensis, C novakii, C. oleophila, C. oregonensis, C. palmyrana, C. paludigena, C. parapsilosis, C. pararugosa, C. pelliculosa, C. peltata, C. periphelosum, C. petrohuensis, C. pignaliae, C. pintolopesii, C. pinus, C. placentae, C. polymorpha, C. populi, C. pseudotropicalis, C. psychrophila, C. pulcherrima, C. punica, C. quercitrusa, C. quercuum, C. railenensis, C. ralunensis, C. reukaufii, C. rhagii, C. rugopelliculosa, C. rugosa, C. saitoana, C. sake, C. salmanticensis, C. santamariae, C. santjacobensis, C. savonica, C. schatavii, C. sequanensis, C. shehatae, C. silvae, C. silvanorum, C. silvicultrix, C. solani, C. sonorensis, C. sophiae-reginae, C. sorboxylosa, C. spandovensis, C. sphaerica, C. stellata, C. stellatoidea, C. succiphila, C. sydowiorum, C. tanzawaensis, C. tenuis, C. tepae, C. terebra, C. torresii, C. tropicalis, C. tsuchiyae, C. tsukubaensis, C. utilis, C. valdiviana, C. valida, C. vanderwaltii, C. vartiovaarai, C. versatilis, C. vini, C. viswanathii, C. wickerhamii, C. xestobii, C. zeylanoides.

Nach einer weiteren bevorzugten Ausführungsform wird die Anhaftung von Pilzen der Spezies Rhodotorula spp., Cryptococcus spp., Exophilia spp. , Hormoconis spp. vermindert.

Besonders bevorzugt wird erfindungsgemäß die Anhaftung der medizinisch relevanten Formen von Candida vermindert, beispielsweise von C. albicans, C. boidinii, C. catenulata, C. ciferii, C. dubliniensis, C. glabrata, C. guilliermondii, C. haemulonii, C. kefyr, C. krusei, C. lipolytica, C. lusitaniae, C. norvegensis, C. parapsilosis, C. pulcherrima, C. rugosa, C. tropicalis, C. utilis, C. viswanathii. Insbesondere bevorzugt sind C. albicans, C. stellatoidea, C. tropicalis, C. glabrata und C. parapsilosis. Die Mycel-Form von Candida wird als humanpathogene Form des Pilzes betrachtet. Eine Verminderung der Anhaftung von Candida beispielsweise an Textilien oder Kunststoffen vermindert das Risiko der Reinfektion, ohne die Bildung von Resistenzen zu erhöhen.

Unter Schimmelpilzen sind gemäß der vorliegenden Erfindung solche Pilze zu verstehen, die ihren Lebensraum im Boden, auf Lebens- und/oder Futtermitteln oder in konzentrierten Nährlösungen haben, ein typisches Mycel bilden und ihre Nährstoffe aus organischen Substanzen gewinnen, die sie dadurch zersetzen (saprobiontische bzw. saprophytische Lebensweise). Des weiteren vermehren sie sich überwiegend ungeschlechtlich durch Sporen (insbesondere Sporangiosporen oder Konidien) und bilden, wenn überhaupt, nur sehr kleine sexuelle Fortpflanzungsorgane aus.

Dazu sind zum Beispiel Spezies aus den Klassen Ascomycota, Basidiomycota, Deuteromycota und Zygomycota zu zählen, insbesondere alle Spezies der Gattungen Aspergillus, Penicillium, Cladosporium und Mucor sowie Stachybotrys, Phoma, Alternaria, Aureobasidium, Ulocladium, Epicoccum, Stemphyllium, Paecilomyces, Trichoderma, Scopulariopsis, Wallemia, Botrytis, Verticillium und Chaetonium.

Zu den Ascomycota gehören hier insbesondere alle Spezies der Gattungen Aspergillus, Penicillium und Cladosporium. Diese Pilze bilden Sporen aus, die bei Kontakt mit der Haut oder den Atemwegen ein stark allergieauslösendes Potential aufweisen. Zu den Basidomycota ist beispielsweise Cryptococcus neoformans zu zählen. Zu den Deuteromycota sind alle als Schimmelpilze bekannten Gattungen zu zählen, insbesondere solche, die durch das Fehlen eines sexuellen Stadiums nicht den Klassen Ascomycota, Basidiomycota oder Zygomycota zugeordnet werden.

Nichtionische Tenside sind besonders bevorzugt zur Verminderung der Anhaftung aller Spezies der Gattung Aspergillus an Oberflächen geeignet, ganz besonders bevorzugt der Spezies, die ausgewählt sind aus Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae var. gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae und Aspergillus wentii. Besonders bevorzugt wird die Anhaftung von Aspergillus flavus und Apsergillus nidulans vermindert bzw. im wesentlichen ganz verhindert.

Unter keratinophilen Pilzen sind solche Haut- und/oder Haarpilze zu verstehen, die in verhornter Haut und deren Anhangsgebilden (insbesondere Haaren und/oder Nägeln) wachsen. Insbesondere sind darunter Dermatophyten und alle Spezies der Gattung Malassezia zu verstehen. Unter Dermatophyten sind erfindungsgemäß insbesondere alle Spezies der Gattungen Trichophyton, Microsporum und Epidermophyton zu verstehen.

Der keratinophile Pilz Malassezia, ein Hefepilz, gilt als Erreger von vermehrter Schuppenbildung der Haut, beispielsweise auf dem Kopf (Haarschuppen). Außerdem wird dieser Organismus als Auslöser der Hautkrankheit Pityriasis versicolor betrachtet. Insbesondere ist es daher von Vorteil, die Adhäsion von Malassezia, insbesondere die der Spezies M. furfur (auch bekannt unter dem Namen Pityrosporum ovale), M. pachydermatis, M. sympodialis und/oder M. globosa zu vermindern bzw. im wesentlichen zu verhindern.

Gemäß einer bevorzugten Ausführungsform sind die keratinophilen Pilze ausgewählt aus Trichophyton mentagrophytes, T. rubrum, T. asteroides, T. concentrium, T. equinum, T. meginii, T. gallinae, T. tonsurans, T. schoenleinii, T. terrestre, T. verrucosum, T. violaceum, Microsporum canis, Microsporum audounii, M. gypseum, Epidermophyton flossocum, Malassezia furfur, M. sympodialis, M. globosa und M. pachydermatis.

Nach einer weiteren bevorzugten Ausführungsform wird durch die Verwendung von nichtionischen Tensiden die Anhaftung von Dermatophyten an Oberflächen vermindert. Insbesondere sind die Dermatophyten ausgewählt aus Trichophyton mentagrophytes, T. rubrum, T. asteroides, T. concentrium, T. equinum, T. meginii, T. gallinae, T. tonsurans, T. schoenleinii, T. terrestre, T. verrucosum, T. violaceum, Microsporum canis, Microsporum audounii, M. gypseum und Epidermophyton flossocum.

Gemäß einer weiteren Ausführungsform wird durch die Verwendung von nichtionischen Tensiden die Anhaftung von Algen, von human-, tier- und/oder pflanzenpathogenen Viren sowie von Bakteriophagen vermindert.

Algen sind ein- bis vielzellige, verschieden gefärbte, primär photoautotrophe Pflanzen bzw. photoautotrophe Bakterien von meist thallophytischer Organisation, deren Gameten und Sporen bildende Organe in der Regel einzellig sind und eventuell Hüllen aus sterilen Zellen besitzen. Algen werden nach ihrer Pigmentzusammensetzung in Grün-, Rot-, Blau- und Braunalgen unterschieden, wobei vor allem Grün- und Blaualgen an Fassaden und Baustoffen relevant sind. Die relevanten Vertreter der Blaualgen (Cyanobacteria) sind aus den Gattungen Anabaena, Anacystis, z.B. Anacystis montana, Gloeocapsa, Lyngbia, Nostoc, Oscillatoria, z.B. Oscillatoria lutea, Phormidium, Schiszothrix und Scytonema. Gattungen der Grünalgen (Chlorophyta) sind beispielsweise Chlorella, Choricystis, Chlamydomonas, Chlorococcum, Stichcoccus, insb. Stichcoccus bacillaris, Ulothrix und Trentepholia, insb. Trentepholia odorata. Erfindungsgemäß kann nun die Anhaftung von Algen auf Oberflächen insbesondere in sehr feuchten Räumen sowie Aquarien, aber auch auf solchen Oberflächen, die der Witterung ausgesetzt sind, wie z.B. Baustoffen, darunter insbesondere Dichtstoffe bzw. Versiegelungen, durch die Verwendung von nichtionischen Tensiden vermindert werden.

### Bevorzugte Gegenstände

Bevorzugte Gegenstände der vorliegenden Erfindung sind Baustoffe und Bauhilfsstoffe, die zur Verminderung der Anhaftung von Mikroorganismen an Oberflächen nichtionische Tenside, insbesondere fluorierte nichtionische Tenside, enthalten. Weitere bevorzugte Gegenstände der vorliegenden Erfindung sind Baustoffe und Bauhilfsstoffe ausgewählt aus Klebe-, Dichtungs- und Spachtelmassen, die mit nichtionischen Tensiden beschichtet sind und/oder mit diesen ausgerüstet wurden, wobei die nichtionischen Tenside vorzugsweise nicht kovalent an die Materialien gebunden sind und wobei die nichtionischen Tenside ausgewählt sind aus:
a) Anlagerungsprodukten von 5 bis 15 Ethylenoxid-Einheiten an C6-C12-Alkylphenole,
b) Anlagerungsprodukten von 10 bis 22 Ethylenoxid-Einheiten an Fettalkohole mit 10 bis 22 C-Atomen,
c) Anlagerungsprodukten von zwischen 5 und 11 Ethylenoxid-Einheiten an Fettalkohole mit 10 bis 20 C-Atomen,
d) Anlagerungsprodukten von zwischen 8 und 12 Ethylenoxid-Einheiten an Fettalkohole mit 18 und 26 C-Atomen,
e) Anlagerungsprodukten von zwischen 3 und 7 Ethylenoxid-Einheiten und zwischen 2 und 6 Propylenoxid-Einheiten an Fettalkohole mit zwischen 10 und 16 C-Atomen.

Die Ausrüstung von Verpackungen, Oberflächen, Textilien, Pelze, Felle, Papier oder Leder mit nichtionischen Tensiden erfolgt in dem Fachmann bekannter Weise, beispielsweise durch Eintauchen in eine geeignet konzentrierte Lösung eines erfindungsgemäßen Mittels oder durch Besprühen mit einer solchen Lösung. So können beispielsweise auch Kunstwerke auf Papier, Pergament, Holz und/oder Leinwand vor dem Befall durch Mikroorganismen, insbesondere Schimmelbefall, geschützt bzw. von diesem befreit werden. Die Ausrüstung der Filtermedien, Baustoffe oder Bauhilfsstoffe erfolgt beispielsweise durch Aufbringen, besonders bevorzugt jedoch durch mechanisches Einarbeiten der nichtionischen Tenside oder einer geeignet konzentrierten Lösung der nichtionischen Tenside auf bzw. in die Filtermedien, Baustoffe oder Bauhilfsstoffe. Die nichtionischen Tenside können so in die betreffenden Materialien vor deren Einsatz mechanisch, z.B. durch Verrühren, eingearbeitet werden oder sie können alternativ auch bereits während der Herstellung der Materialien in diese eingearbeitet werden.

Vorzugsweise sind die erfindungsgemäß ausgerüsteten Baustoffe oder Bauhilfsstoffe ausgewählt unter Klebe-, Dichtungs-, Spachtel- und Anstrichmassen, Kunststoffen, Lacken, Farben, Putz, Mörtel, Estrich, Beton, Isoliermaterialien sowie Grundierungen. Besonders bevorzugte Baustoffe oder Bauhilfsstoffe sind Fugendichtungsmassen (bspw. silikonhaltige Fugendichtungsmassen), Tapetenkleister, Putz, Teppichfixierer, Silikonkleber, Fliesenkleber.

Dichtungsmassen und insbesondere Fugendichtungsmassen enthalten typischerweise organische Polymere sowie in vielen Fällen mineralische oder organische Füllstoffe und sonstige Additive.

Geeignete Polymere sind beispielsweise thermoplastische Elastomere, wie in der DE 3602526 beschrieben, vorzugsweise Polyurethane und Acrylate. Geeignete Polymere sind auch in den Offenlegungsschriften DE 3726547, DE 4029504 und DE 4009095 sowie in DE 19704553 und DE 4233077 genannt, auf die hiermit in vollem Umfang Bezug genommen wird.

Die erfindungsgemäßen Dichtungsmassen (Dichtstoffe bzw. Dichtstoffmischungen) enthalten bevorzugt 0,001 - 3,0 Gew.-% nichtionische Tenside. Besonders bevorzugt sind Mengen zwischen 0,01 und 1,0 Gew.-%.

Erfindungsgemäß kann die Ausrüstung der erfindungsgemäßen Dichtstoffe sowohl im ungehärteten oder bei unter 60 °C gehärteten Zustand erfolgen. Dichtstoffe sind im erfindungsgemäßen Zusammenhang Materialien gemäß DIN EN 26927, insbesondere solche, die plastisch oder elastisch als Dichtstoffe aushärten. Die erfindungsgemäßen Dichtstoffe können alle für die entsprechenden Dichtungsmassen typischen Zusatzstoffe, wie z.B. typische Verdickungsmittel, verstärkende Füllstoffe, Vernetzer, Vernetzungskatalysatoren, Pigmente, Haftmittel oder sonstige Volumenextender enthalten. Das Aufbringen bzw. Einbringen der nichtionischen Tenside kann durch Eindispergieren in dem Fachmann bekannter Weise z.B. durch die Verwendung von Dispergiereinrichtungen, Kneter, Planetenmischer usw., unter Ausschluss von Feuchtigkeit und Sauerstoff sowohl in die fertige als auch in Teile dieser Dichtungsmassen bzw. zusammen mit einer oder mehreren Komponenten der Dichtungsmassen erfolgen.

Selbst die Behandlung von bereits ausgehärteten, vernetzten Dichtungsmassenoberflächen kann durch Aufbringen von Lösungen bzw. Suspensionen der erfindungsgemäß verwendeten Substanz durchgeführt werden, indem der Wirkstoff durch Quellung bzw. Diffusion in die Dichtungsmasse transportiert wird.

Erfindungsgemäß einsetzbare Dichtstoffe können sowohl auf Silikon-, Urethan- als auch auf Acrylbasis oder etwa auf MS-Polymerbasis hergestellt werden. Dichtstoffe auf Urethanbasis sind bspw. in Ullmann's Encyclopedia of Industrial Chemistry (8. Auflage 2003, Kapitel 4) sowie US 4,417,042 offenbart. Silikondichtstoffe sind dem Fachmann bekannt, bspw. aus der EP 0 118 030 A, EP 0 316 591 A, EP 0 327 847 A, EP 0 553 143 A, DE 195 49 425 A und US 4,417,042. Beispiele für Acryldichtstoffe sind u.a. in der WO 01/09249 oder der US 5,077,360 offenbart. Beispiele für Dichtstoffe auf MS-Polymerbasis sind beispielsweise in der EP 0 824 574, US 3,971,751, US 4,960,844, US 3,979,344, US 3,632,557, DE 4029504, EP 601 021 oder der EP 370 464 offenbart.

In einer besonders bevorzugten Ausführungsform handelt es sich bei der Fugendichtungsmasse um eine Fugendichtungsmasse auf Silikon-Basis, insbesondere ausgewählt aus Acetat-, Alkoxy-, Oxim-, Benzamid- und Aminsilikonen. Die Fungedichtungsmasse enthält hierbei vorzugsweise als Polyorganosiloxane und als Organosilikonverbindungen mit hydrolysierbaren Gruppen Verbindungen wie sie in der Patentschrift US 5,378,406 beschrieben werden in den dort angegebenen Mengen, deren diesbezügliche Offenbarung hiermit zum Gegenstand dieser Patentanmeldung gemacht wird.

Insbesondere sind bei Raumtemperatur vernetzende Systeme, wie bspw. in der EP 0 327 847 oder US 5,077,360 beschrieben, bevorzugt. Dabei kann es sich um ein- oder mehrkomponentige Systeme handeln, wobei in den mehrkomponentigen Systemen Katalysator und Vernetzer getrennt vorliegen können (beispielsweise offenbart in den Patentschriften US 4,891,400 und US 5,502,144), oder andere sogenannte Silikon RVT 2K-Systeme, insbesondere platinfreie Systeme.

Besonders bevorzugt sind sogenannte Einkomponentensysteme, die alle Inhaltsstoffe zum Aufbau einer Dichtungsmasse enthalten, unter Abschluß von Luftfeuchtigkeit und/oder Luftsauerstoff gelagert werden und am Einsatzort unter Reaktion mit dem Luftsauerstoff und/oder der Luftfeuchtigkeit aushärten. Besonders bevorzugt sind die sogenannten Silikon-Neutralsysteme, in denen die Umsetzung von Vernetzern mit dem Wasser der Umgebungsluft nicht zu korrosiven, sauren, basischen oder geruchsintensiven Spaltprodukten führt. Beispiele für solche Systeme sind in der DE 195 49 425, der US 4,417,042 oder der EP 0 327 847 offenbart.

Die Dichtungsmassen und insbesondere Fugendichtungsmassen können wäßrige oder organische Lösungsmittel enthalten. Als organische Lösungsmittel kommen Kohlenwasserstoffe wie Cyclohexan, Toluol oder auch Xylol oder Petrolether in Frage. Weitere Lösungsmittel sind Ketone wie Methylbutylketon oder Chlorkohlenwasserstoffe.

Weiterhin können die Dichtungsmassen noch weitere kautschukartige Polymere enthalten. Hier kommen relativ niedermolekulare, handelsübliche Typen von Polyisobutylen, Polyisopren oder auch Polybutadienstyrol in Frage. Auch die Mitverwendung von abgebautem Naturkautschuk oder von Neoprenkautschuk ist möglich. Hier können auch bei Raumtemperatur noch fließfähige Typen eingesetzt werden, welche häufig als "Flüssigkautschuk" bezeichnet werden.

Die erfindungsgemäßen Dichtungsmassen können verwendet werden, um die verschiedensten Materialien miteinander zu verbinden bzw. abzudichten. Hier ist in erster Linie an die Verwendung auf Beton, auf Glas, auf Putz und/oder Emaille sowie Keramik und Porzellan gedacht. Aber auch das Verbinden bzw. Abdichten von Formteilen bzw. Profilen aus Aluminium, Stahl, Zink oder auch aus Kunststoffen wie PVC oder Polyurethanen oder Acrylharzen ist möglich. Schließlich sei das Abdichten von Holz oder Holzmaterialien mit den verschiedensten anderen Werkstoffen erwähnt.

Die Standfestigkeit von Fugendichtungsmassen wird in der Regel durch Zusatz von feinteiligen Feststoffen — auch Füllstoffe genannt — erzielt. Diese lassen sich in solche organischer und solche anorganischer Art unterscheiden. Als anorganische Füllstoffe können beispielsweise Kieselsäure/Siliciumdioxid (gecoatet oder ungecoatet), Kreide — gecoatet oder ungecoatet — und/oder Zeolithe bevorzugt sein. Letztere können zudem auch als Trockenmittel fungieren. Als organischer Füllstoff kommt z.B. PVC- Pulver in Betracht. Die Füllstoffe tragen im allgemeinen wesentlich dazu bei, dass die Dichtungsmasse nach der Anwendung einen notwendigen inneren Halt besitzt, so dass ein Auslaufen oder Ausbuchten der Dichtungsmasse aus senkrechten Fugen verhindert wird. Die genannten Zusatz- bzw. Füllstoffe lassen sich in Pigmente und thixotropierende Füllstoffe, auch verkürzt als Thixotropiermittel bezeichnet, einteilen.

Als Thixotropierungsmittel eignen sich die bekannten Thixotropierungsmittel wie Bentone, Kaoline oder auch organische Verbindungen wie hydriertes Rizinusöl bzw. Derivate desselben mit mehrfunktionellen Aminen oder die Umsetzungsprodukte von Stearinsäure oder Rizinolsäure mit Ethylendiamin. Als besonders günstig hat sich die Mitverwendung von Kieselsäure, insbesondere von Kieselsäure aus der Pyrolyse erwiesen. Außerdem kommen als Thixotropiermittel im wesentlichen quellfähige Polymerpulver in Betracht. Beispiele sind hierfür Polyacrylnitril, Polyurethan, Polyvinylchlorid, Polyacrylsäureester, Polyvinylalkohole, Polyvinylacetate sowie die entsprechenden Copolymerisate. Besonders gute Ergebnisse lassen sich mit feinteiligem Polyvinylchloridpulver erhalten. Neben den Thixotropierungsmitteln können auch noch zusätzlich Haftvermittler eingesetzt werden wie etwa Mercaptoalkylsilan. Hier hat es sich als zweckmäßig erwiesen, ein Monomercaptoalkyltrialkoxysilan einzusetzen. Handelsüblich ist beispielsweise das Mercaptopropyltrimethoxysilan.

Die Eigenschaften einer Fugendichtungsmasse lassen sich noch weiter verbessern, wenn dem als Thixotropiermittel verwendeten Kunststoffpulver weitere Komponenten zugesetzt werden. Dabei handelt es sich um Stoffe, die unter die Kategorie der für Kunststoffe angewendeten Weichmacher bzw. Quellmittel und Quellhilfsmittel fallen.

Es kommen z. B. Weichmacher, insbesondere für die Dichtungsmassen auf Urethan- bzw. Acrylbasis aus der Klasse der Phthalsäureester in Betracht. Beispiele für anwendbare Verbindungen aus dieser Substanzklasse sind Dioctylphthalat, Dibutylphthalat und Benzylbutylphthalat. Weitere geeignete Substanzklassen sind Chlorparaffine, Alkylsulfonsäureester etwa der Phenole oder Kresole sowie Fettsäureester.

Für die Silikondichtungsmassen sind als Weichmacher Silikonöle, besonders bevorzugt Polydimethylsiloxane, sowie Kohlenwasserstoffe und/oder deren Gemische, von denen besonders Kohlenwasserstoffe bzw. deren Gemische mit einem Siedepunkt von größer 200°C, insbesondere größer 230 °C, gut geeignet.

Als Quellhilfsmittel sind solche niedermolekularen organischen Substanzen einsetzbar, die mit dem Polymerpulver und dem Weichmacher mischbar sind. Derartige Quellhilfsmittel lassen sich den einschlägigen Kunststoff- und Polymer-Handbüchern für den Fachmann entnehmen. Als bevorzugte Quellhilfsmittel für Polyvinylchloridpulver dienen Ester, Ketone, aliphatische Kohlenwasserstoffe, aromatische Kohlenwasserstoffe sowie aromatische Kohlenwasserstoffe mit Alkylsubstituenten.

Als Pigmente und Farbstoffe werden die für diese Verwendungszwecke bekannten Substanzen wie Titandioxid, Eisenoxide und Ruß verwendet

Zur Verbesserung der Lagerstabilität werden bekanntermaßen den Dichtungsmassen Stabilisatoren wie Benzoylchlorid, Acetylchlorid, Toluolsulfonsäuremethylester, Carbodiimide und/oder Polycarbodiimide zugesetzt. Als besonders gute Stabilisatoren haben sich Olefine mit 8 bis 20 Kohlenstoffatomen erwiesen. Neben der stabilisierenden Wirkung können diese auch Aufgaben von Weichmachern bzw. Quellmitteln erfüllen. Bevorzugt werden Olefine mit 8 bis 18 Kohlenstoffatomen, insbesondere wenn die Doppelbindung in 1,2-Stellung angeordnet ist. Beste Ergebnisse erhält man, wenn die Molekülstruktur dieser Stabilisatoren linear ist.

Durch die erfindungsgemäße Verwendung von nichtionischen Tensiden zur Verminderung der Anhaftung von Mikrooganismen, insbesondere von Schimmelpilzen an Oberflächen, umgeht man das Problem der Resistenzbildung aufgrund biozider Wirkstoffe. Bei der Anwendung in schimmelgefährdeten Bau- und Bauhilfsstoffen, insbesondere in Dichtungsmassen und besonders bevorzugt in Fugendichtungsmassen werden durch die Verminderung der Anhaftung von Schimmelpilzen an Oberflächen mehrere erwünschte Effekte erzielt: es werden Verfärbungen durch pigmentierte Schimmelpilze verhindert, es wird die Ausbreitung des Schimmelbefalls verzögert und es wird die Allergenbelastung vermindert.

Eine weitere bevorzugte Ausführungsform der vorliegenden Erfindung sind Tapetenkleber, enthaltend vorzugsweise 0,000001 bis 3 Gew.-% nichtionische Tenside. Bei den Tapetenklebern kann es sich hierbei um Tapetenkleister aus wässrigen Lösungen von Hydrokolloiden wie Methylcellulose, Methylhydroxypropylcellulose oder wasserlöslichen Stärkederivaten oder etwa um wässrige Dispersionen von filmbildenden Hochmolekularen wie Polyvinylacetat, insbesondere in Verbindung mit den bereits erwähnten Cellulose- und Stärkederivaten, handeln.

Als Filtermedien können alle bekannten Arten eingesetzt werden, solange sie für den Einsatz in Wasser- oder Luftfilteranlagen, insbesondere für Klimaanlagen oder Raumluftbefeuchter, geeignet sind. Insbesondere sind Filtermaterialien aus Cellulose, Glasfasern, PVC-Fasern, Polyesterfasern, Polyamidfasern, insbesondere Nylonfasern, Vliesstoffen, Sintermaterialien und Membranfilter zu nennen.

Die Konzentration der zur Verminderung der Anhaftung von Mikrooganismen an Oberflächen einzusetzenden nichtionischen Tenside in den erfindungsgemäßen Mitteln kann durch den Fachmann in einem breiten Bereich variiert werden, abhängig von den Einsatzbedingungen der Mittel.

Die erfindungsgemäßen Mittel werden nach üblichen und dem Fachmann bekannten Rezepturen hergestellt. Die nichtionischen Tenside können sowohl den bereits fertig zubereiteten Mitteln zugegeben, aber ebenso auch während des Herstellungsprozesses zugesetzt werden.

### Wasch- und Reinigungsmittel

Für Waschmittel wurde bereits beschrieben, dass hier nichtionische Tenside zur Reinigung der Wäsche eingesetzt werden können. Allerdings ist bisher ein antiadhäsiver Effekt noch nicht beschrieben worden.

Ein weiterer Gegenstand der Erfindung ist daher die Verwendung von nichtionischen Tensiden in Wasch- und/oder Reinigungsmitteln zur Verminderung der Anhaftung von Mikroorganismen an mit diesen Wasch- und/oder Reinigungsmitteln behandelten Gegenständen, dadurch gekennzeichnet, dass die nichtionischen Tenside ausgewählt sind aus:
a) Anlagerungsprodukten von 5 bis 15 Ethylenoxid-Einheiten an C6-C12-Alkylphenole,
b) Anlagerungsprodukten von 10 bis 22 Ethylenoxid-Einheiten an Fettalkohole mit 10 bis 22 C-Atomen,
c) Anlagerungsprodukten von zwischen 5 und 11 Ethylenoxid-Einheiten an Fettalkohole mit 10 bis 20 C-Atomen,
d) Anlagerungsprodukten von zwischen 8 und 12 Ethylenoxid-Einheiten an Fettalkohole mit 18 und 26 C-Atomen,
e) Anlagerungsprodukten von zwischen 3 und 7 Ethylenoxid-Einheiten und zwischen 2 und 6 Propylenoxid-Einheiten an Fettalkohole mit zwischen 10 und 16 C-Atomen.

Solche Wasch- und Reinigungsmittel können ohne Belastung der Abwässer relativ geringe Mengen der Substanzen enthalten. Da sie in konzentrierter Form eingesetzt und auf die entsprechend wirksamen Konzentrationen in der Waschlauge verdünnt werden, müssen die Wirkstoffe in entsprechend höherer Konzentration eingesetzt werden. Üblich sind Verdünnungen der Wasch- und Reinigungsmittel mit Wasser zwischen 1:40 und 1:200.

Nichtionische Tenside können erfindungsgemäß auch zu Reinigungsmitteln, die zum Säubern harter Oberflächen, wie zum Beispiel von Böden, Kacheln, Fliesen, Kunststoffen sowie anderen harten Oberflächen im Haushalt, in öffentlichen sanitären Anlagen, in Schwimmbädern, Saunen, Sportanlagen oder in Arzt- oder Massagepraxen zugegeben werden.

Neben den pathogenen Mikroorganismen (insbesondere Pilze und Bakterien) sind auf solchen Oberflächen insbesondere Pseudomonas aeruginosa, Salmonelle spec., Actinobacteria (insbesondere Brachybacterium spec.), alpha-Proteobacteria (insbesondere Agrobacterium spec.), beta-Proteobacteria (insbesondere Nitrosomonas spec., Aquabacterium spec., Hydrogenophaga), gamma- Proteobacteria (insbesondere Stenotrophomonas spec., Xanthomonas spec (campestris)).

Unter Wasch- und Reinigungsmitteln werden im erfindungsgemäßen Zusammenhang im weitesten Sinn tensidhaltige Zubereitungen in fester Form (Partikel, Pulver usw.), halbfester Form (Pasten usw.), flüssiger Form (Lösungen, Emulsionen, Suspensionen, Gele usw.) und gasähnlicher Form (Aerosole usw.) verstanden, die im Hinblick auf eine vorteilhafte Wirkung bei der Anwendung neben den fluorierten nichtionischen Tensiden auch weitere Tenside enthalten können, üblicherweise neben weiteren Komponenten, die für den jeweiligen Anwendungszweck üblich sind. Beispiele für solche tensidhaltige Zubereitungen sind tensidhaltige Waschmittelzubereitungen, tensidhaltige Reinigungsmittel für harte Oberflächen, oder tensidhaltige Aviviermittelzubereitungen, die jeweils fest oder flüssig sein können, jedoch auch in einer Form vorliegen können, die feste und flüssige Komponenten oder Teilmengen der Komponenten nebeneinander umfasst.

Die Wasch- und Reinigungsmittel können üblicherweise enthaltende Inhaltsstoffe enthalten, wie anionische, nichtionische, kationische und amphotere Tenside, anorganische und organische Buildersubstanzen, spezielle Polymere (beispielsweise solche mit Cobuildereigenschaften), Schauminhibitoren, Farbstoffe und ggf. zusätzliche Duftstoffe (Parfums), Bleichmittel (wie beispielsweise Peroxo-Bleichmittel und Chlor-Bleichmittel), Bleichaktivatoren, Bleichstabilisatoren, Bleichkatalysatoren, Enzyme und Vergrauungsinhibitoren, ohne dass die Inhaltsstoffe auf diese Substanzgruppen beschränkt sind. Häufig sind wichtige Inhaltsstoffe dieser Zubereitungen auch Waschhilfsmittel, für die beispielhaft und nicht beschränkend optische Aufheller, UV-Schutzsubstanzen, sog. Soil Repellents, also Polymere, die einer Wiederanschmutzung von Fasern entgegenwirken, verstanden werden. Die einzelnen Substanzgruppen werden im weiteren näher erläutert.

Für den Fall, dass die Zubereitungen zumindest zum Teil als Formkörper vorliegen, können auch Binde- und Desintegrationshilfsmittel enthalten sein.

Als weitere Tenside können neben den fluorhaltigen nichtionischen Tensiden weitere nichtionische Tenside sowie anionische, zwitterionische und kationische Tenside in den Waschmitteln enthalten sein.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, in Betracht, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von 2-Sulfofettsäuren (Estersulfonate), z.B. die 2-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. In Wasch- und Reinigungsmitteln sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Wasch- und Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen.

Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Bevorzugt sind die Natrium- oder Kaliumsalze, insbesondere die Natriumsalze. Die Tenside können ebenfalls in Form ihrer Magnesiumsalze eingesetzt werden.

Im Rahmen der vorliegenden Erfindung sind solche Mittel bevorzugt, die 5 bis 50 Gew.-%, vorzugsweise 7,5 bis 40 Gew.-% und insbesondere 15 bis 25 Gew.-% eines oder mehrerer anionischer Tensid(e), enthalten.

Neben den zuvor genannten nichtionischen Tensiden können in den Waschmitteln weitere nichtionische Tenside enthalten sein wie beispielsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, sowie Mischungen aus C₁₂₋₁₄-Alkohol mit 3 EO und C₁₂₋₁₈-Alkohol- mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhaft eingesetzt werden kann, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen steht und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Bevorzugt eingesetzt werden lineare Alkylpolyglucoside, also Alkylpolyglycoside, in denen der Polyglycosylrest ein Glucoserest und der Alkylrest ein n-Alkylrest ist.

Die erfindungsgemäßen tensidhaltigen Zubereitungen können bevorzugt Alkylpolyglycoside enthalten, wobei Gehalte der für Wasch-, Spül- oder Reinigungszwecke vorgesehenen Zubereitungen an APG von über 0,2 Gew.-%, bezogen auf die gesamte Zubereitung, bevorzugt sind. Besonders bevorzugte tensidhaltige Zubereitungen enthalten APG in Mengen von 0,2 bis 10 Gew.-%, vorzugsweise in Mengen von 0,2 bis 5 Gew.-% und insbesondere in Mengen von 0,5 bis 3 Gew.-%.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (I), 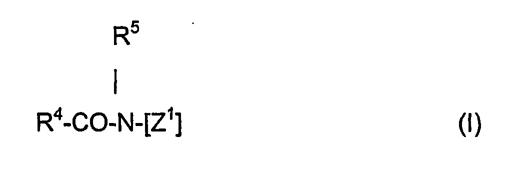 in der R⁴CO für einen aliphatischen Acylrest mit 6 bis 22 Kohlenstoffatomen, R⁵ für Wasserstoff, einen Alkyl- oder Hydroxyalkylrest mit 1 bis 4 Kohlenstoffatomen und [Z¹] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (II), 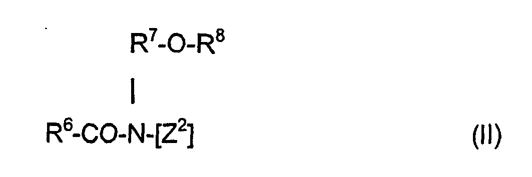 in der R⁶ für einen linearen oder verzweigten Alkyl- oder Alkenylrest mit 7 bis 12 Kohlenstoffatomen, R⁷ für einen linearen, verzweigten oder cyclischen Alkylrest oder einen Arylrest mit 2 bis 8 Kohlenstoffatomen und R⁸ für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z²] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z²] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise, wie in der WO-A-95/07331 beschrieben, durch Umsetzung mit Fettsäuremethylestern in Gegenwart eines Alkoxids als Katalysator in die gewünschten Polyhydroxyfettsäureamide überführt werden.

Weiterhin kann es bevorzugt sein, neben anionischen und nichtionischen Tensiden auch kationische Tenside einzusetzen.

Als textilweichmachende Substanzen sind insbesondere kationische Tenside zu nennen. Beispiele kationische Tenside sind insbesondere quartäre Ammoniumverbindungen, kationische Polymere und Emulgatoren.

Geeignete Beispiele sind quartäre Ammoniumverbindungen der Formeln (III) und (IV) 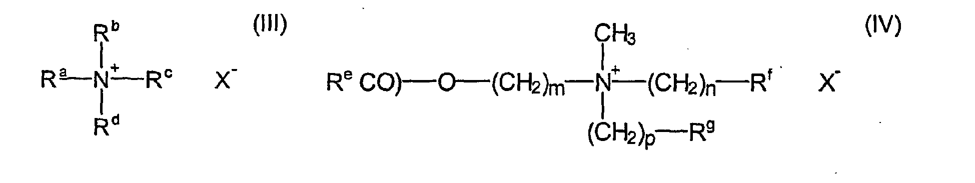 wobei in (IV) R^{a} und R^{b} für einen acyclischen Alkylrest mit 12 bis 24 Kohlenstoffatomen, R^{c} für einen gesättigten C₁-C₄ Alkyl- oder Hydroxyalkylrest steht, R^{d} entweder gleich R^{a}, R^{b} oder R^{c} ist oder für einen aromatischen Rest steht. X⁻ steht entweder für ein Halogenid-, Metho-sulfat-, Methophosphat- oder Phosphation sowie Mischungen aus diesen. Beispiele für kationische Verbindungen der Formel (III) sind Didecyldimethylammoniumchlorid, Ditalgdimethylammoniumchlorid oder Dihexadecylammoniumchlorid.

Verbindungen der Formel (IV) sind sogenannte Esterquats. Esterquats zeichnen sich durch eine hervorragende biologische Abbaubarkeit aus. Hierbei steht R^{e} für einen aliphatischen Acylrest mit 12 bis 22 Kohlenstoffatomen mit 0, 1, 2 oder 3 Doppelbindungen; R^{f} steht für H, OH oder O(CO)R^{h}, R^{g} steht unabhängig von R^{f} für H, OH oder O(CO)Rⁱ, wobei R^{h} und Rⁱ unabhängig voneinander jeweils für einen aliphatischen Acylrest mit 12 bis 22 Kohlenstoffatomen mit 0, 1, 2 oder 3 Doppelbindungen steht. m, n und p können jeweils unabhängig voneinander den Wert 1, 2 oder 3 haben. X⁻ kann entweder ein Halogenid-, Methosulfat-, Methophosphat- oder Phosphation sowie Mischungen aus diesen sein. Bevorzugt sind Verbindungen, die für R^{f} die Gruppe O(CO)R^{h} und für R^{c} und R^{h} Alkylreste mit 16 bis 18 Kohlenstoffatomen enthalten. Besonders bevorzugt sind Verbindungen, bei denen R^{g} zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere größer als 70 : 30 haben. Handelsübliche Beispiele sind die von Stepan unter der Marke Stepantex^{®} vertriebenen Methylhydroxyalkyldialkoyloxyalkylammoniummethosulfate oder die unter Dehyquart^{®} bekannten Produkte von Cognis bzw. die unter Rewoquat^{®} bekannten Produkte von Goldschmidt-Witco. Weitere bevorzugte Verbindungen sind die Diesterquats der Formel (V), die unter dem Namen Rewoquat^{®} W 222 LM bzw. CR 3099 erhältlich sind und neben der Weichheit auch für Stabilität und Farbschutz sorgen.

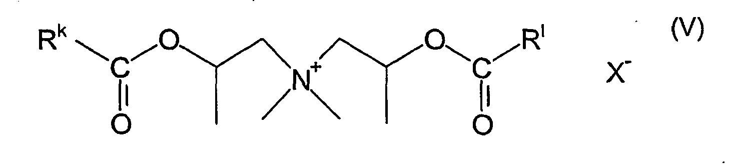

R^{k} und R^{l} stehen dabei unabhängig voneinander jeweils für einen aliphatischen Acylrest mit 12 bis 22 Kohlenstoffatomen mit 0, 1, 2 oder 3 Doppelbindungen.

Neben den oben beschriebenen quartären Verbindungen können auch andere bekannte Verbindungen eingesetzt werden, wie beispielsweise quartäre Imidazoliniumverbindungen der Formel (VI), 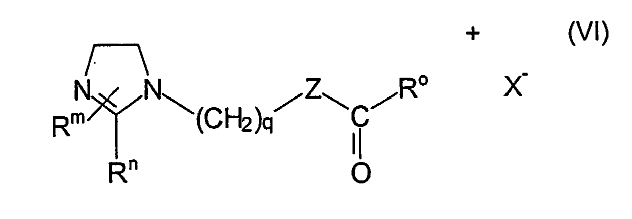 wobei R^{m} für H oder einen gesättigten Alkylrest mit 1 bis 4 Kohlenstoffatomen, Rⁿ und R^{o} unabhängig voneinander jeweils für einen aliphatischen, gesättigten oder ungesättigten Alkylrest mit 12 bis 18 Kohlenstoffatomen, Rⁿ alternativ auch für O(CO)R^{p} stehen kann, wobei R^{p} einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen.

Weitere geeignete quartäre Verbindungen sind durch Formel (VII) beschrieben, 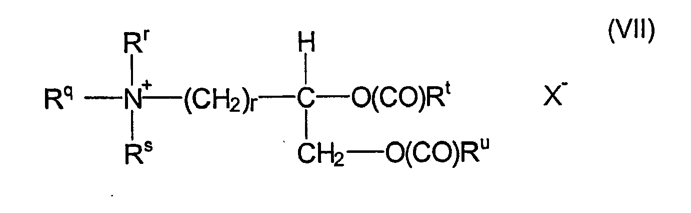 wobei R^{q}, R^{r} und R^{s} unabhängig voneinander für eine C₁₋₄-Alkyl-, Alkenyl- oder Hydroxyalkylgruppe steht, R^{t} und R^{u} jeweils unabhängig ausgewählt eine C₈₋₂₈-Alkylgruppe darstellt und r eine Zahl zwischen 0 und 5 ist.

Neben den Verbindungen der Formeln III bis VII können auch kurzkettige, wasserlösliche, quartäre Ammoniumverbindungen eingesetzt werden, wie Trihydroxyethylmethylammonium-methosulfat oder die Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet.

Weitere erfindungsgemäß verwendbare kationische Verbindungen stellen die quaternisierten Proteinhydrolysate dar.

Zu den geeigneten kationischen Polymeren zählen die Polyquaternium-Polymere, wie sie im CTFA Cosmetic Ingredient Dictionary (The Cosmetic, Toiletry und Fragrance, Inc., 1997), insbesondere die auch als Merquats bezeichneten Polyquaternium-6-, Polyquaternium-7-, Polyquaternium-10-Polymere (Ucare Polymer IR 400; Amerchol), Polyquaternium-4-Copolymere, wie Pfropfcopolymere mit einen Cellulosegerüst und quartären Ammoniumgruppen, die über Allyldimethylammoniumchlorid gebunden sind, kationische Cellulosederivate, wie kationisches Guar, wie Guarhydroxypropyltriammoniumchlorid, und ähnliche quaternierte Guar-Derivate (z. B. Cosmedia Guar, Hersteller: Cognis GmbH), kationische quartäre Zuckerderivate (kationische Alkylpolyglucoside), z. B. das Handelsprodukt Glucquat^{®}100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosilicon-polymere und Copolymere,

Ebenfalls einsetzbar sind polyquaternierte Polymere (z. B. Luviquat Care von BASF) und auch kationische Biopolymere auf Chitinbasis und deren Derivate, beispielsweise das unter der Handelsbezeichnung Chitosan^{®} (Hersteller: Cognis) erhältliche Polymer.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) Abil^{®}-Quat 3270 und 3272 (Hersteller: Goldschmidt-Rewo; diquartäre Polydimethylsiloxane, Quaternium-80), sowie Siliconquat Rewoquat^{®} SQ 1 (Tegopren^{®} 6922, Hersteller: Goldschmidt-Rewo).

Ebenfalls einsetzbar sind Verbindungen der Formel (VIII), 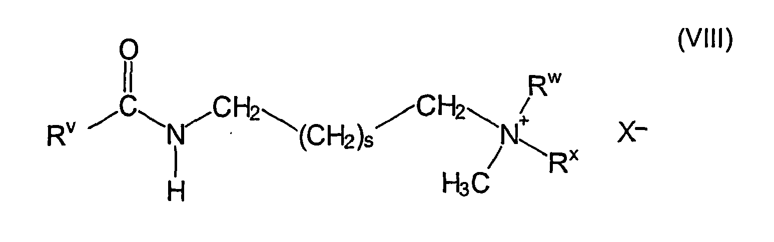 die Alkylamidoamine in ihrer nicht quaternierten oder, wie dargestellt, ihrer quaternierten Form, sein können. R^{v} kann ein aliphatischer Acylrest mit 12 bis 22 Kohlenstoffatomen mit 0, 1, 2 oder 3 Doppelbindungen sein. s kann Werte zwischen 0 und 5 annehmen. R^{w} und R^{x} stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid^{®}S 18 erhältliche Stearylamidopropyldimethylamin oder das unter der Bezeichnung Stepantex^{®} X 9124 erhältliche 3-Talgamidopropyltrimethylammonium-methosulfat, die sich neben einer guten konditionierenden Wirkung auch durch farbübertragungsinhibierende Wirkung sowie speziell durch ihre gute biologische Abbaubarkeit auszeichnen.

Werden kationische Tenside eingesetzt, so sind sie in den Zubereitungen bevorzugt in Mengen von 0,01 bis 10 Gew.-%, insbesondere von 0,1 bis 3,0 Gew.-% enthalten.

Der Gesamttensidgehalt kann in den erfindungsgemäßen Mitteln zwischen 5 und 50 Gew.-%, bevorzugt zwischen 10 und 35 Gew.-% liegen.

Neben den Tensiden sind Gerüststoffe die wichtigsten Inhaltsstoffe von Wasch- und Reinigungsmitteln. In den erfindungsgemäßen tensidhaltigen Zubereitungen können üblicherweise in Wasch- und Reinigungsmitteln eingesetzte Gerüststoffe enthalten sein, insbesondere also Zeolithe, Silicate, Carbonate, organische Cobuilder und - wo keine ökologischen Vorurteile gegen ihren Einsatz bestehen - auch die Phosphate.

Geeignete kristalline, schichtförmige Natriumsilicate besitzen die allgemeine Formel NaMSiₓO₂ₓ₊₁ ·H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung EP-A-0 164 514 beschrieben. Bevorzugte kristalline Schichtsilikate der angegebenen Formel sind solche, in denen M für Natrium steht und x die Werte 2 oder 3 annimmt. Insbesondere sind sowohl β- als auch δ-Natriumdisilicate Na₂Si₂O₅ · yH₂O bevorzugt, wobei β-Natriumdisilicat beispielsweise nach dem Verfahren erhalten werden kann, das in der internationalen Patentanmeldung WO-A-91/08171 beschrieben ist.

Einsetzbar sind auch amorphe Natriumsilicate mit einem Modul Na₂O : SiO₂ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Sogenannte röntgenamorphe Silicate, welche ebenfalls eine Löseverzögerung gegenüber den herkömmlichen Wassergläsern aufweisen, werden beispielsweise in der deutschen Patentanmeldung DE-A- 44 00 024 beschrieben. Die Produkte weisen mikrokristalline Bereiche der Größe 10 bis einige Hundert nm auf, wobei Werte bis max. 50 nm und insbesondere bis max. 20 nm bevorzugt sind. Insbesondere bevorzugt sind verdichtete/kompaktierte amorphe Silicate, compoundierte amorphe Silicate und übertrocknete röntgenamorphe Silicate.

Ein gegebenenfalls eingesetzter feinkristalliner, synthetischer und gebundenes Wasser enthaltender Zeolith ist vorzugsweise Zeolith A und/oder P. Als Zeolith des P-Typs wird Zeolith MAP (z. B. Handelsprodukt: Doucil A24 der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist in Waschmitteln auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern deren Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von tensidhaltigen Zubereitungen gemäß der Erfindung. Insbesondere sind in diesem Zusammenhang Citronensäure, Bernsteinsäure, Glutarsäure, Adipinsäure, Gluconsäure und beliebige Mischungen von diesen zu nennen.

Als Builder sind weiter polymere Polycarboxylate geeignet. Dies sind beispielsweise die Alkalimetallsalze der Polyacrylsäure oder der Polymethacrylsäure, beispielsweise solche mit einer relativen Molekülmasse von 500 bis 70.000 g/mol.

Bei den für polymere Polycarboxylate angegebenen Molmassen handelt es sich im Rahmen der vorliegenden Erfindung um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsäuren gemessenen Molmassen sind in der Regel deutlich höher als die im Rahmen der vorliegenden Erfindung angegebenen Molmassen.

Geeignete Polymere sind insbesondere Polyacrylate, die bevorzugt eine Molmasse von 2.000 bis 20.000 g/mol aufweisen. Aufgrund ihrer überlegenen Löslichkeit können aus dieser Gruppe wiederum die kurzkettigen Polyacrylate bevorzugt sein, die Molmassen von 2.000 bis 10.000 g/mol, besonders bevorzugt von 3.000 bis 5.000 g/mol, aufweisen.

Geeignet sind weiterhin copolymere Polycarboxylate, insbesondere solche der Acrylsäure mit Methacrylsäure oder der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Die (co-)polymeren Polycarboxylate können entweder als Pulver oder als wäßrige Lösung eingesetzt werden. Der Gehalt an (co-)polymeren Polycarboxylaten in den erfindungsgemäßen Wasch- und Reinigungsmitteln beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, Allyloxybenzolsulfonsäure und Methallylsulfonsäure als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder Salze der Acrylsäure und der 2-Alkylallylsulfonsäure sowie Zucker-Derivate enthalten.

Weiter bevorzugte Copolymere sind solche, die als Monomere vorzugsweise Acrolein und Acrylsäure/Acrylsäuresalze bzw. Acrolein und Vinylacetat enthalten.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die zum Teil neben Co-Builder-Eigenschaften auch eine bleichstabilisierende Wirkung aufweisen.

Weitere geeignete Buildersubstanzen sind Polyacetale, die durch Umsetzung von Dialdehyden mit Polyolcarbonsäuren erhalten werden können, die 5 bis 7 Kohlenstoffatome und mindestens 3 Hydroxygruppen aufweisen. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose-Äquivalent (DE) im Bereich von 0,5 bis 40, insbesondere von 2 bis 30, bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2.000 bis 30.000 g/mol. Ein bevorzugtes Dextrin ist in der britischen Patentanmeldung 9419 091 beschrieben.

Bei den oxidierten Derivaten derartiger Dextrine handelt es sich um deren Umsetzungsprodukte mit Oxidationsmitteln, die in der Lage sind, mindestens eine Alkoholfunktion des Saccharidrings zur Carbonsäurefunktion zu oxidieren. Ebenfalls geeignet ist ein oxidiertes Oligosaccharid, wobei ein an C₆ des Saccharidrings oxidiertes Produkt besonders vorteilhaft sein kann.

Auch Oxydisuccinate und andere Derivate von Disuccinaten, vorzugsweise Ethylendiamindisuccinat sind weitere geeignete Co-Builder. Dabei wird Ethylendiamin-N,N'-disuccinat (EDDS) bevorzugt in Form der Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Co-Builder sind beispielsweise acetylierte Hydroxycarbonsäuren bzw. deren Salze, welche gegebenenfalls auch in Lactonform vorliegen können und welche mindestens 4 Kohlenstoffatome und wenigstens eine Hydroxygruppe sowie maximal zwei Säuregruppen enthalten.

Eine weitere Substanzklasse mit Co-Builder-Eigenschaften stellen die Phosphonate dar. Dabei handelt es sich insbesondere um Hydroxyalkan- bzw. Aminoalkanphosphonate. Unter den Hydroalkanphosphonaten ist das 1-Hydroxyethan-1,1-diphosphonat (HEDP) von besonderer Bedeutung als Co-Builder. Es wird vorzugsweise als Natriumsalz eingesetzt, wobei das Dinatriumsalz neutral und das Tetranatriumsalz alkalisch (pH = 9) reagiert. Als Aminoalkanphosphonate kommen vorzugsweise Ethylendiamintetramethylenphosphonat (EDTMP), Diethylentriaminperitamethylenphosphonat (DTPMP) sowie deren höhere Homologe in Frage. Sie werden vorzugsweise in Form der neutralreagierenden Natriumsalze, z.B. als Hexanatriumsalz der EDTMP bzw. als Hepta- und Octanatriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die erfindungsgemäßen tensidhaltigen Zubereitungen auch Bleiche enthalten, bevorzugt sein, Aminoalkanphosphonate, insbesondere DTPMP, einzusetzen oder Mischungen aus den genannten Phosphonaten zu verwenden.

Darüber hinaus können alle Verbindungen, die in der Lage sind, Komplexe mit Erdalkalimetallionen zu bilden, als Co-Builder eingesetzt werden.

Unter den als Bleichmittel dienenden, in Wasser H₂O₂ liefernden Verbindungen haben das Natriumperborat-tetrahydrat und das Natriumperborat-monohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Werden Reinigungs- oder Bleichmittel-Zubereitungen für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesiummonoperphthalat; (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamido-peroxycapronsäure, N-Nonenylamidoperadipinsäure und N-Nonenylamidoper-succinate; und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäue) können eingesetzt werden.

Um beim Waschen oder Reinigen bei Temperaturen von 60 °C und darunter eine verbesserte Bleichwirkung zu erreichen, können Bleichaktivatoren in die tensidhaltigen Zubereitungen eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole; insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren in die tensidhaltigen Zubereitungen eingearbeitet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen tensidhaltigen Zubereitungen kann beispielsweise etwa 0,1 bis 5 Gew.-%, vorzugsweise 0,1 bis etwa 2 Gew.-%, betragen.

Eine bevorzugte Gruppe geeigneter Additive sind optische Aufheller. Verwendet werden können hier die in Waschmitteln üblichen optischen Aufheller. Beispiele für optische Aufheller sind Derivate von Diaminostilbendisulfonsäure bzw. deren Alkalimetallsalze. Geeignet sind z. B. Salze der 4, 4'-Bis(2-anilino-4-morpholino1,3,5-triazinyl-6-amino)stilben-2,2'-disulfonsäure oder gleichartig aufgebaute Verbindungen, die anstelle der Morpholino-Gruppe eine Diethanol-amino-Gruppe, eine Methylamino-Gruppe, eine Anilino-Gruppe oder eine 2-Methoxyethylamino-Gruppe tragen. Weiterhin können Aufheller vom Typ der substituierten Diphenylstyryle in den Teil-Portionen (waschaktiven Zubereitun-gen) der erfindungsgemäßen tensidhaltigen Zubereitungen enthalten sein, z. B. die Alkalisalze des 4,4'-Bis(2-sulfostyryl-)diphenyls, 4,4'-Bis(4-chlor-3-sulfostyryl-)diphenyls oder 4-(4-Chlorstyryl-)4'-(2-sulfostyryl-)diphenyls. Auch Gemische der vorgenannten Aufheller können verwendet werden.

Eine weitere erfindungsgemäß bevorzugte Gruppe von Additiven sind UV-Schutz-Substanzen. UV-Absorber können auf die behandelten Textilien aufziehen und die Lichtbeständigkeit der Fasern und/oder die Lichtbeständigkeit des sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/öder 4-Stellung. Weiterhin sind auch substituierte Benzotriazole, wie beispielsweise das wasserlösliche Benzolsulfonsäure-3-(2H-benzotriazol-2-yl)-4-hydroxy-5-(methylpropyl)-mononatriumsalz (Cibafast^{®} H), in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen 3-Benzylidencampher bzw. 3-Benzylidennorcampher und dessen Derivate, z.B. 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Die UV-Absorber werden üblicherweise in Mengen von 0,01 Gew.-% bis 5 Gew.-%, vorzugsweise von 0,03 Gew.-% bis 1 Gew.-%, eingesetzt.

Eine weitere erfindungsgemäß bevorzugte Gruppe von Additiven sind Farbstoffe, insbesondere wasserlösliche oder wasserdispergierbare Farbstoffe. Bevorzugt sind hier Farbstoffe, wie sie zur Verbesserung der optischen Produktanmutung in den erfindungsgemäßen Wasch-, Spül-, Reinigungs- und Textilbehandlungsmitteln üblicherweise eingesetzt werden. Die Auswahl derartiger Farbstoffe bereitet dem Fachmann keine Schwierigkeiten, insbesondere da derartige übliche Farbstoffe eine hohe Lagerstabilität und Unempfindlichkeit gegenüber den übrigen Inhaltsstoffen der waschaktiven Zubereitungen und gegen Licht sowie keine ausgeprägte Substantivität gegenüber Textilfasern haben, um diese nicht anzufärben. Die Farbstoffe sind erfindungsgemäß in den Wasch- und/oder Reinigungsmitteln gemäß der Erfindung in Mengen von unter 0,01 Gew.-% zugegen.

Eine weitere Klasse von Additiven, die erfindungsgemäß den Wach- und/oder Reinigungsmitteln zugesetzt werden kann, sind Polymere. Unter diesen Polymeren kommen zum einen Polymere in Frage, die beim Waschen oder Reinigen bzw. Spülen Cobuilder-Eigenschaften zeigen, also zum Beispiel Polyacrylsäuren, auch modifizierte Polyacrylsäuren oder entsprechende Copolymere. Eine weitere Gruppe von Polymeren sind Polyvinylpyrrolidon und andere Vergrauungsinhibitoren, wie Copolymere von Polyvinylpyrrolidon, Cellulose-Ether und dergleichen. Weiterhin kommen als Polymere bevorzugt auch sogenannte Soil Repellents in Frage, wie sie nachfolgend im einzelnen beschrieben werden.

Als weitere erfindungsgemäße Zusätze können die Wasch- und Reinigungsmittel auch sog. Soil Repellents enthalten, also Polymere, die auf Fasern aufziehen, die Öl- und Fettauswaschbarkeit aus Textilien positiv beeinflussen und damit einer Wiederanschmutzung gezielt entgegenwirken. Dieser Effekt wird besonders deutlich, wenn ein Textil verschmutzt wird, das bereits vorher mehrfach mit einem erfindungsgemäßen Wasch- oder Reinigungsmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxy-Gruppen von 15 bis 30 Gew.-% und an Hydroxypropoxy-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Insbesondere wenn es sich um flüssige oder gelförmige Zubereitungen handelt, können diese auch Lösungsmittel enthalten. Beispiele für geeignete Lösungsmittel sind ein- oder mehrwertige Alkohole mit 1 bis 4 C-Atomen. Bevorzugte Alkohole sind Ethanol, 1,2-Propandiol, Glycerin sowie deren beliebigen Gemische. Die Lösungsmittel können in flüssigen Zubereitungen in einer Menge von 2 bis 12 Gew.-%, bezogen auf die fertige Zubereitung, enthalten sein.

Die genannten Additive werden den Wasch- und/oder Reinigungsmitteln in Mengen bis höchstens 30 Gew.-%, vorzugsweise 2 bis 20 Gew.-%, zugesetzt.

Diese Aufzählung von Wasch- und Reinigungsmittelinhaltsstoffen, die in den erfindungsgemäßen Wasch-, Spül- oder Reinigungsmittel vorkommen können, ist keineswegs abschließend, sondern gibt lediglich die wesentlichen typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Nach einer besonderen Ausführungsform sind insbesondere flüssige oder feste Waschmittel bevorzugt. Ebenfalls besonders bevorzugt sind Wasch- und Reinigungsmittel, die für die Feinwäsche bzw. schonende Behandlung von empfindlichen Textilien geeignet sind.

Insbesondere sind auch Textilpflegemittel, insbesondere Textilnachbehandlungsmittel, bevorzugt Textilkonditioniermittel, Weichspüler oder Trocknertücher geeignet, die Patchouliöl, Patchoulialkohol und/oder dessen Derivate enthalten.

Je nach gewünschtem Verwendungszweck können weitere Inhaltsstoffe eingesetzt werden. Weichspülerzusammensetzungen für die Spülbadavivage sind im Stand der Technik breit beschrieben. Üblicherweise enthalten diese Zusammensetzungen als Aktivsubstanz eine kationische quartäre Ammoniumverbindung, die in Wasser dispergiert wird. Je nach Gehalt der fertigen Weichmacherzusammensetzung an Aktivsubstanz spricht man von verdünnten, anwendungsfertigen Produkten (Aktivsubstanzgehalte unter 7 Gew.-%) oder sogenannten Konzentraten (Aktivsubstanzgehalt über 7 Gew.-%). Wegen des geringeren Volumens und den damit gleichzeitig verringerten Verpackungs- und Transportkosten besitzen die Textilweichmacherkonzentrate Vorteile aus ökologischer Sicht und haben sich im Markt mehr und mehr durchgesetzt. Aufgrund der Einarbeitung von kationischen Verbindungen, die nur eine geringe Wasserlöslichkeit aufweisen, liegen übliche Weichspülerzusammensetzungen in Form von Dispersionen vor, besitzen ein milchigtrübes Aussehen und sind nicht durchscheinend. Aus Gründen der Produktästhetik kann es aber auch gewünscht sein, dem Verbraucher durchscheinende, klare Weichspüler zur Verfügung zu stellen, die sich optisch von den bekannten Produkten abheben.

Als textilweichmachende Aktivsubstanz enthalten erfindungsgemäße Weichspüler vorzugsweise kationische Tenside, die bereits weiter oben ausführlich beschrieben wurden. Besonders bevorzugt enthalten diese erfindungsgemäße Mittel sogenannte Esterquats. Während es eine Vielzahl möglicher Verbindungen aus dieser Substanzklasse gibt, werden erfindungsgemäß mit besonderem Vorzug Esterquats eingesetzt, die sich durch Umsetzung von Trialkanolaminen mit einer Mischung aus Fettsäuren und Dicarbonsäuren, gegebenenfalls nachfolgende Alkoxylierung des Reaktionsproduktes und Quaternierung in an sich bekannter Weise herstellen lassen, wie es in der DE 195 39 846 beschrieben ist.

Die auf diese Weise hergestellten Esterquats eignen sich in hervorragender Weise zur Herstellung erfindungsgemäßer Portionen, die als Weichspüler eingesetzt werden können. Da je nach Wahl des Trialkanolamins, der Fettsäuren und der Dicarbonsäuren sowie des Quaternierungsmittels eine Vielzahl geeigneter Produkte hergestellt und in den erfindungsgemäßen Mitteln eingesetzt werden kann, ist eine Beschreibung der erfindungsgemäß vorzugsweise einzusetzenden Esterquats über ihren Herstellungsweg präziser als die Angabe einer allgemeinen Formel.

Die genannten Komponenten, die miteinander zu den vorzugsweise einzusetzenden Esterquats reagieren, können in variierenden Mengenverhältnissen zueinander eingesetzt werden. Im Rahmen der vorliegenden Erfindung sind Weichspüler bevorzugt, in denen ein Umsetzungsprodukt von Trialkanolaminen mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten ist. Besonders bevorzugt ist dabei die Verwendung von Triethanolamin, so daß weitere bevorzugte Weichspüler der vorliegenden Erfindung ein Umsetzungsprodukt von Triethanolamin mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten.

Als Fettsäuren können im Reaktionsgemisch zur Herstellung der Esterquats sämtliche aus pflanzlichen oder tierischen Ölen und Fetten gewonnenen Säuren verwendet werden. Dabei kann im Rekationsgemisch als Fettsäure durchaus auch eine bei Raumtemperatur nicht-feste, d.h. pastöse bis flüssige, Fettsäure eingesetzt werden.

Die Fettsäuren können unabhängig von ihrem Aggregatzustand gesättigt oder ein- bis mehrfach ungesättigt sein. Selbstverständlich können nicht nur "reine" Fettsäuren eingesetzt werden, sondern auch die bei der Spaltung aus Fetten und Ölen gewonnenen technischen Fettsäuregemische, wobei diese Gemische aus ökonomischer Sicht wiederum deutlich bevorzugt sind.

So lassen sich in den Reaktionsmischungen zur Herstellung der Esterquats für die erfindungsgemäßen klaren wäßrigen Weichspüler beispielsweise einzelne Spezies oder Gemische folgender Säuren einsetzen: Caprylsäure, Pelargonsäure, Caprinsäure, Laurinsäure, Myristinsäure, Palmitinsäure, Stearinsäure, Octadecan-12-ol-säure, Arachinsäure, Behensäure, Lignocerinsäure, Cerotinsäure, Melissinsäure, 10-Undecensäure, Petroselinsäure, Petroselaidinsäure, Ölsäure, Elaidinsäure, Ricinolsäure, Linolaidinsäure, α- und β-Eläosterainsäure, Gadoleinsäure, Erucasäure, Brassidinsäure. Selbstverständlich sind auch die Fettsäuren mit ungerader Anzahl von C-Atomen einsetzbar, beispielsweise Undecansäure, Tridecansäure, Pentadecansäure, Heptadecansäure, Nonadecansäure, Heneicosansäure, Tricosansäure, Pentacosansäure, Heptacosansäure.

Im Rahmen der vorliegenden Erfindung ist die Verwendung von Fettsäuren der Formel XIII im Reaktionsgemisch zur Herstellung der Esterquats bevorzugt, so daß bevorzugte Weichspüler ein Umsetzungsprodukt von Trialkanolaminen mit einer Mischung aus Fettsäuren der Formel IX,

R¹-CO-OH (IX)

in der R1-CO- für einen aliphatischen, linearen oder verzweigten Acylrest mit 6 bis 22 Kohlenstoffatomen und 0 und/oder 1, 2 oder 3 Doppelbindungen steht und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Als Dicarbonsäuren, die sich zur Herstellung der in den erfindungsgemäßen Mitteln einzusetzenden Esterquats eignen, kommen vor allem gesättigte oder ein- bzw. mehrfach ungesättigte α,□Dicarbonsäuren in Betracht. Beispielhaft seien hier die gesättigten Spezies Oxalsäure, Malonsäure, Bernsteinsäure, Glutarsäure, Adipinsäure, Pimelinsäure, Korksäure, Azelainsäure, Sebacinsäure, Undecan- und Dodecansäure, Brassylsäure, Tetra- und Pentadecansäure, Thapisäure sowie Hepta-, Octa- und Nonadecansäure, Eicosan- und Heneicosansäure sowie Phellogensäure genannt. Vorzugsweise im Reaktionsgemisch eingesetzt werden dabei Dicarbonsäuren, die der allgemeinen Formel XIII folgen, so daß erfindungsgemäße Mittel bevorzugt sind, die ein Umsetzungsprodukt von Trialkanolaminen mit einer Mischung aus Fettsäuren und Dicarbonsäuren der Formel X,

HO-OC-[X]-CO-OH (X)

in der X für eine gegebenenfalls hydroxysubstituierte Alkylengruppe mit 1 bis 10 Kohlenstoffatomen steht, im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Unter der Vielzahl der herstellbaren und erfindungsgemäß einsetzbaren Esterquats haben sich wiederum solche besonders bewährt, in denen das Alkanolamin Treithanolamin und die Dicarbonsäure Adipinsäure ist. Somit sind im Rahmen der vorliegenden Erfindung Mittel besonders bevorzugt, die ein Umsetzungsprodukt von Triethanolamin mit einer Mischung aus Fettsäuren und Adipinsäure im molaren Verhältnis 1:5 bis 5:1, vorzugsweise 1:3 bis 3:1, das anschließend in an sich bekannter Weise quaterniert wurde, in Mengen von 2 bis 60, vorzugsweise 3 bis 35 und insbesondere 5 bis 30 Gew.-% in den Mitteln enthalten

Die erfindungsgemäßen Mittel können - und abhängig davon, ob sie als Textilwaschmittel, Waschhilfsmittel oder Weichspüler formuliert werden - auch mit weiteren Zusatznutzen ausgestattet werden. Hier sind beispielsweise farbübertragungsinhibierende Zusammensetzungen, Mittel mit "Anti-Grau-Formel", Mittel mit Bügelerleichterung, Mittel mit besonderer Duftfreisetzung, Mittel mit verbesserter Schmutzablösung bzw. Verhinderung von Wiederanschmutzung, antibakterielle Mittel, UV-Schutzmittel, farbauffrischende Mittel usw. formulierbar. Einige Beispiele werden nachstehend erläutert:

Da textile Flächengebilde, insbesondere aus Reyon, Zellwolle, Baumwolle und deren Mischungen, zum Knittern eigen können, weil die Einzelfasern gegen Durchbiegen, Knicken. Pressen und Quetschen quer zur Faserrichtung empfindlich sind, können die erfindungsgemäßen Mittel synthetische Knitterschutzmittel enthalten. Hierzu zählen beispielsweise synthetische Produkte auf der Basis von Fettsäuren, Fettsäureestern. Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Ein erhöhter Tragekomfort kann aus der zusätzlichen Verwendung von Antistatika resultieren, die den erfindungsgemäßen Mitteln zusätzlich beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (bzw. Stearyl-) dimethylbenzylammoniumchloride eignen sich als Antistatika für Textilien bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten Textilien und zur Erleichterung des Bügelns der behandelten Textilien können in den erfindungsgemäßen Mitteln beispielsweise Silikonderivate eingesetzt werden. Diese verbessern zusätzlich das Ausspülverhalten der erfindungsgemäßen Mittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25°C im Bereich zwischen 100 und 100.000 Centistokes, wobei die Silikone in Mengen zwischen 0,2 und 5 Gew.-%, bezogen auf das gesamte Mittel eingesetzt werden können.

Schließlich können die erfindungsgemäßen Mittel auch UV-Absorber enthalten, die auf die behandelten Textilien aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

### Kosmetische Zusammensetzungen

Ein weiterer Gegenstand der vorliegenden Erfindung ist die Verwendung von nichtionischen Tensiden in kosmetischen Zusammensetzungen zur Verminderung der Adhäsion von Mikroorganismen, dadurch gekennzeichnet, dass die nichtionischen Tenside ausgewählt sind aus:
a) Anlagerungsprodukten von 5 bis 15 Ethylenoxid-Einheiten an C6-C12-Alkylphenole,
b) Anlagerungsprodukten von 10 bis 22 Ethylenoxid-Einheiten an Fettalkohole mit 10 bis 22 C-Atomen,
c) Anlagerungsprodukten von zwischen 5 und 11 Ethylenoxid-Einheiten an Fettalkohole mit 10 bis 20 C-Atomen,
d) Anlagerungsprodukten von zwischen 8 und 12 Ethylenoxid-Einheiten an Fettalkohole mit 18 und 26 C-Atomen,
e) Anlagerungsprodukten von zwischen 3 und 7 Ethylenoxid-Einheiten und zwischen 2 und 6 Propylenoxid-Einheiten an Fettalkohole mit zwischen 10 und 16 C-Atomen.

Die erfindungsgemäßen kosmetischen Zubereitungen können neben den erfindungsgemäßen Wirkstoffen beispielsweise auch Wirkstoffe enthalten, die die Adhäsion von Pilzen verhindern wie sie beispielsweise in WO 03/051124 beschrieben werden. Ferner kann der Einsatz der erfindungsgemäßen Wirkstoffe in Kombination mit antimikrobiellen, insbesondere antibakteriellen, antimykotischen und/oder antiseptischen Wirkstoffen und/oder in Kombination mit adstringierenden Stoffen erfolgen.

Bei den antimykotischen Wirkstoffen handelt es sich hierbei vorzugsweise um solche, die zur Behandlung bei Pilzbefall, insbesondere im Falle von Candidosen, bereits üblicherweise angewendet werden. Hierbei kann es sich insbesondere um Antimykotika vom Polyen-Typ, vor allem um Nystatin, Amphotericin B oder Natamycin, und/oder um Antimykotika vom Azol-Typ, vor allem um Miconazol, Clotrimazol oder Ketokonazol, handeln. Durch Kombination mit anderen antimykotischen Wirkstoffen kann die Entfernung der Pilze gegebenenfalls beschleunigt werden, wobei vorzugsweise ein synergistischer Effekt auftritt. Bei Verwendung von anti-mikrobiellen Wirkstoffen können diese vorteilhafterweise in niedrigeren Konzentrationen verwendet werden als bei der bisherigen Behandlung üblich.

Bei den adstringierenden Stoffen kann es sich insbesondere um Aluminiumsalze, Phenolkondensationsprodukte (künstliche Gerbstoffe), Naturstoffe und gerbstoffhaltige Drogen handeln.

In einer erfindungsgemäß besonders bevorzugten Ausführungsform handelt es sich bei den kosmetischen Zubereitungen um solche zur topischen Applikation. auf die Haut und deren Anhangsgebilde und/oder zur Applikation auf die Schleimhaut, insbesondere im oralen oder genitalen Bereich, bzw. zur intertriginösen Applikation. Im folgenden werden diese Zubereitungen als "Hautbehandlungsmittel" bezeichnet.

Bei dem Hautbehandlungsmittel kann es sich hierbei insbesondere um eine Lotion, eine Creme, eine Salbe, eine Paste, ein Öl, ein Gel, ein Puder, ein Spray bzw. Aerosol, eine Lösung, insbesondere alkoholische Lösung, bzw. Tinktur, um einen feuchten Verband, einen Okklusionsverband, ein Pflaster, ein Stiftpräparat, ein Haarbehandlungs- oder Haarpflegemittel, insbesondere ein Haarshampoo, eine Haarlotion, eine Haarkur oder ein Haarwasser, ein Schaumbad, ein Duschbad oder ein Fußbad handeln.

Der physiologische Träger der Hautbehandlungsmittel umfasst vorzugsweise ein oder, in beliebiger Kombination, mehrere Hilfsstoffe, wie sie üblicherweise in solchen Zubereitungen verwendet werden, wie z.B. Fette, Öle, Überfettungsmittel, Wachse, Silikone, Emulgatoren, Dispergiermittel, Perlglanzwachse, Alkohole, Polyole, Konsistenzgeber, Stabilisatoren, Verdickungsmittel, Quellmittel, Hydrotrope bzw. anfeuchtende und/oder feuchthaltende Substanzen, Polymere, Tenside, Weichmacher, Schaumbremsen, Alkalinisierungs- oder Acidifizierungsmittel, Enthärter, Adsorbentien, Lichtschutzmittel, Elektrolyte, Sequestrierungsmittel, organische Lösungsmittel, Konservierungsmittel, keimhemmende Wirkstoffe, insbesondere Fungizide oder Bakterizide, Antioxidantien, biogene Wirkstoffe, Vitamine, Proteinhydrolysate, Mono-, Oligo- und Polysaccharide, Enzyminhibitoren, insbesondere MMP1-inhibierende Substanzen, Desodorantien bzw. Geruchsabsorber, Antitranspirantien, Antischuppenmittel, α-Hydroxy- und α-Ketocarbonsäuren, Duftstoffe, Farbstoffe und/oder Pigmente.

Die erfindungsgemäßen Hautbehandlungsmittel liegen bei topischer Verabreichung vorteilhafterweise in Form einer flüssigen oder festen Öl-in-Wasser-Emulsion, Wasserin-Öl-Emulsion, Mehrfach-Emulsion, Mikroemulsion, PIT-Emulsion oder Pickering-Emulsion, in Form eines Hydrogels, eines alkhoholischen Gels, eines Lipogels, in Form einer ein- oder mehrphasigen Lösung, eines Schaumes, einer Salbe, eines Pflasters, einer Suspension, eines Puders oder einer Mischung mit mindestens einem als medizinischen Klebstoff geeigneten Polymer vor. Die erfindungsgemäßen Hautbehandlungsmittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer weiteren erfindungsgemäß besonders bevorzugten Ausführungsform handelt es sich bei den kosmetischen Zubereitungen um solche zur oralen Applikation, wobei der Zielort der Applikation der Mund selbst ist. In einer bevorzugten Ausführungsform wird hierbei eines der zuvor beschriebenen Hautbehandlungsmittel verwendet, wobei die Zusammensetzung so gewählt wird, dass es sich bei dem Präparat um eine Mundcreme, eine Salbe, eine Tinktur oder um eine Suspension handelt. Der Begriff "pharmazeutische Zubereitungen zur oralen Applikation" umfasst hierbei auch Prothesenreinigungsmittel, insbesondere Gebissreinigungstabletten.

Des weiteren sind im oralen Bereich Mundwässer, Zahncremes, Tabletten, insbesondere Lutschtabletten sowie Sprays bzw. Aerosole weitere bevorzugte Ausführungsformen.

Bei Teilprothesen bzw. Gebissen eignet sich sowohl die Darreichung als Gebissreinigungstabletten, als auch als Mundspülung bzw. Mundwasser oder als Zahnpasta.

Die erfindungsgemäßen Mund-, Zahn- und/oder Zahnprothesenpflegemittel können beispielsweise als Mundwasser, Gel, flüssige Zahnputzlotion, steife Zahnpaste, Gebissreiniger oder Prothesenhaftcreme vorliegen. Hierzu ist es erforderlich, die erfindungsgemäß verwendeten Stoffe in einen geeigneten Träger einzubringen.

Die erfindungsgemäßen Zahnpasten und Zahngele können als Inhaltsstoffe neben den erfindungsgemäßen Wirkstoffen insbesondere Tenside, Putzkörper, Aromen, Süßungsmittel sowie weitere dem Fachmann bekannte Wirkstoffe enthalten. Als Träger dienen vorzugsweise Wasser und Bindemittel. Ferner können etwa auch Feuchthaltemittel, Konservierungsstoffe, Konsistenzgeber und/oder Farbpigmente enthalten sein.

Bei den erfindungsgemäßen Mundwässer kann es sich um wässrige, insbesondere auch alkoholhaltige, aromatisierte Konzentrate oder auch um gebrauchsfertige Lösungen handeln. Die Mundwässer können neben den erfindungsgemäßen Wirkstoffen insbesondere Tenside, Aromen, Farbstoffe, Fluoride, adstringierende Substanzen, antibakterielle Substanzen und/oder weitere Wirkstoffe enthalten.

Bei den zuvor genannten weiteren Wirkstoffen, die in den Mundbehandlungsmitteln enthalten sein können, kann es sich beispielsweise um eine Fluor-Verbindung, um einen Wirkstoff gegen Plaque-Bakterien, um einen Wirkstoff gegen Zahnsteinbildung, zur Reminalisierung, gegen sensible Zähne oder zum Schutz des Zahnfleischs handeln. Des weiteren kann es sich bei dem weiteren Wirkstoff um einen weiteren Wirkstoff zur Pilzbehandlung, insbesondere Candidosen-Behandlung, handeln.

Weitere übliche Zusätze für die Mund-, Zahn- und/oder Zahnprothesenpflege mittel sind z.B.
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄
- wundheilende und entzündungshemmende Stoffe wie z.B. Allantoin, Harnstoff, Panthenol, Azulen bzw. Kamillenextrakt
- weitere gegen Zahnstein wirksame Stoffe wie z.B. Organophosphonate, z.B. Hydroxyethandiphosphonate oder Azacycloheptandiphosphonat
- Konservierungsstoffe wie z.B. Sorbinsäure-Salze, Natriumbenzoat, Chlorhexidindigluconat, p-Hydroxybenzoesäure oder deren Ester.
- Plaque-Inhibitoren wie z.B. Hexachlorophen, Chlorhexidin, Hexetidin, Triclosan, Bromchlorophen, Phenylsalicylsäureester.

### Beispiele:

### 1. Adhäsionstests mit Schimmelpilzen

Es wurde die Silicondichtmasse DC3390 verwendet. Es wurde 1 Gew.-% Tensid hinzugegeben und im Speedmixer homogenisiert. Getestet wurden die in Tabelle 1 angegebenen Tenside im Vergleich zu Fugendichtungsmasse ohne Zusatz des zu testenden Tensids. Die erhaltene Masse wurde als Film (22 x 22 x 2 mm) verstrichen und an der Luft härten gelassen. Der so erhaltene Prüfkörper wurde mit 70 % Ethanol 10 Minuten desinfiziert, anschließend mit destilliertem Wasser gewaschen und getrocknet. Anschließend wurden die Prüfkörper mit einer Keimsuspension von Aspergillus niger überschichtet und für eine Stunde inkubiert. Danach wurde die Keimsuspension abgesaugt und der Prüfkörper zweimal gewaschen. Die Prüfkörper wurden in sterile Röhrchen, die 5 ml Inaktivierungslösung und Glasperlen enthielten, überführt und auf einem Schüttler für 10 min bei 400 rpm geschüttelt. Um die Anzahl der adhärierten Sporen zu bestimmen, wurden einerseits die Prüfkörper in sterile 6-wellPlatten gelegt und mit Würzeagar mit INT überschichtet, andererseits wurde eine Keimzahlbestimmung der Inaktivierungslösung mittels Plattengussverfahren durchgeführt. Anschließend erfolgte nach 48 Stunden Inkubation bei 25°C die Bestimmung der Anzahl der koloniebildenden Einheiten (KBE). Bei einigen der eingesetzten Substanzen konnte eine deutliche Reduktion der Anzahl koloniebildender Einheiten erreicht werden (Tabelle 1).

### 2. Inkorporationsversuch mit Schimmelpilzen

Zur Überprüfung der fungiziden Wirkung der Substanzen wurde der Inkorporationsversuch mit Aspergillus niger durchgeführt. Es wurden 6 ml flüssiger Malzextraktagar (ca. 50°C) in eine Petrischale mit einem Durchmesser von ca. 6 cm gefüllt. Hierzu wurden jeweils 300 µl Tensid-Wirkstofflösung 10%ig hinzugegeben und homogen, durch Schwenken der Petrischale, verteilt. Somit war die Wirkstoffkonzentration 0,5 % im Agar. Untersucht wurden Polyethylenglycol 600, Polyethylenglycol 2000, Polyethylenglykoldimetylether, Eumulgin B1, Eumulgin B2, Mergital B10, Biodac 2/32, Dehydol 100, Dehydol LT7, Dehypon LS54 sowie die Kieselsäureester von Dehydol LT 7 und Biodac 2/32. Nach Erhärten der Agaroberfläche wurde die Oberfläche mit einer Sporensuspension von Aspergillus niger beaufschlagt. Die Agarplatten wurden bei 25°C inkubiert. Die Auswertung erfolgte durch visuelle Beurteilung des Wachstums im Vergleich zur Kontrolle ohne Wirkstoff. Bei keiner der getesteten Subtanzen war eine Beeinträchtigung des Wachstums zu beobachten (Tabelle 1).

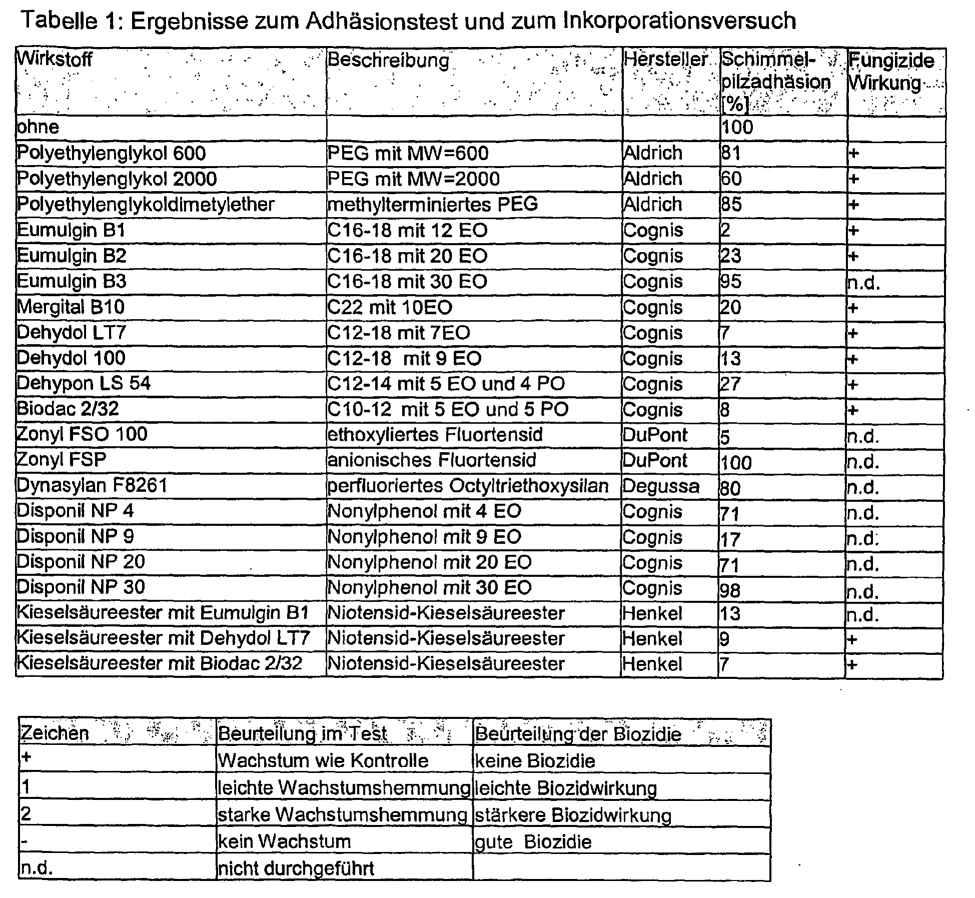

### 3. Flüssigwaschmittel

| Rohstoff | Menge in Gewichtsprozent |
|---|---|
| C₁₂-C₁₈ Fettalkohol + 7 EO (Dehydol LT 7, Cognis) | 15 |
| C₁₂-C₁₄ Fettalkohol C₁₂-C₁₈ Fettalkohol + 7 EO (Dehydol LT 7, Cognis)+ 2 EO-sulfat, Natriumsalz (Texapon N 70, Cognis) | 7 |
| C₈₋₁₈ -Fettsäure geschnitten (Kokosölfettsäure, Edenor K12-18, Cognis) | 8 |
| Natriumcitrat | 1,5 |
| Enzyme | + |
| Farbstoff | + |
| Parfüm | + |
| Nichtionisches Tensid | 0,4 |
| Wasser | Ad 100 |

### 4. Vorportioniertes Flüssigwaschmittel in Polyvinylalkohol-Folie

| Rohstoff | Menge in Gewichtsprozent |
|---|---|
| C₁₂₋₁₄-Fettalkohol+5-EO+4-PO (Marlox MO 154, Sasol) | 25 |
| Dodecylbenzolsulfonat-Isopropylammoniumsalz (LAS-MlPA, Sasol) | 24,5 |
| C₈₋₁₈ -Fettsäure geschnitten (Kokosölfettsäure, Edenor K12-18, Cognis) | 17,5 |
| Ethanol | 3,5 |
| Natriumcitrat | 0,6 |
| Enzyme | 2,0 |
| Wasser | 6,0 |
| Nichtionisches Tensid | 0,6 |
| Farbstoff | + |
| Parfum | + |
| Propylenglycol | ad 100 |

Das vorportionierte Waschmittel wird mit 50 ml dosiert.

### 5. Pulverförmiges Waschmittel

| Rohstoff | Menge in Gewichtsprozent |
|---|---|
| C₁₀-C₁₃-Alkylbenzolsulfonat | 13,3 |
| C₁₂-C₁₈-Alkylsulfat | 5,5 |
| C₁₂-C₁₈-Alkohol mit 7 EO | 5,3 |
| C₁₂-C₁₈-Alkohol mit 4,5 EO | 0,6 |
| Soil Repellent | 0,7 |
| C₁₆-C₁₈ Fettsäure (Edenor ST1 C₁₆-C₁₈, Cognis) | 0,8 |
| Polyethylenglykol Molekulargewicht= 4000 g/mol | 1,8 |
| Phosphonat | 1,0 |
| Polyacrylate | 2,8 |
| Carboxymethylcellulose | 0,9 |
| Polyvinylpyrrolidon | 0,5 |
| Zeolith (wasserfreie Aktivsubstanz) | 32,1 |
| Natriumcarbonat | 4,5 |
| Natriumtricitrat | 3,6 |
| Citronensäure | 3,7 |
| Natriumhydrogencarbonat | 4,9 |
| Natriumsulfat | 3,8 |
| Entschäumer | + |
| Enzyme | + |
| Farbstoff | + |
| Parfüm | + |
| Nichtionisches Tensid | 0,4 |
| Wasser / Salze | Ad 100 |

Das Waschmittel wird mit 75 g dosiert.

Die nichtionischen Tenside können auch als Bestandteil des Parfüms eingearbeitet werden. Sie sind dann in Konzentrationen von 0,1 - 80 Gew.-% im Parfümöl enthalten und werden über das in der Waschmittelrezeptur enthaltende Parfümöl in die Waschflotte eingetragen.

## Patentansprüche

1. Verwendung von nichtionischen Tensiden zur Verminderung der Anhaftung von Mikroorganismen an Oberflächen, **dadurch gekennzeichnet, dass** die nichtionischen Tenside ausgewählt sind aus:
a) Anlagerungsprodukten von 5 bis 15 Ethylenoxid-Einheiten an C6-C12-Alkylphenole,
b) Anlagerungsprodukten von 10 bis 22 Ethylenoxid-Einheiten an Fettalkohole mit 10 bis 22 C-Atomen,
c) Anlagerungsprodukten von zwischen 5 und 11 Ethylenoxid-Einheiten an Fettalkohole mit 10 bis 20 C-Atomen,
d) Anlagerungsprodukten von zwischen 8 und 12 Ethylenoxid-Einheiten an Fettalkohole mit 18 und 26 C-Atomen,
e) Anlagerungsprodukten von zwischen 3 und 7 Ethylenoxid-Einheiten und zwischen 2 und 6 Propylenoxid-Einheiten an Fettalkohole mit zwischen 10 und 16 C-Atomen.

2. Verwendung nach Anspruch 1, **dadurch gekennzeichnet, dass** die Mikroorganismen ausgewählt sind aus der Gruppe der Bakterien und der Pilze.

3. Verwendung nach Anspruch 2, **dadurch gekennzeichnet, dass** die Pilze ausgewählt sind aus Hefen, Schimmelpilzen und/oder keratinophilen Pilzen.

4. Verwendung nach Anspruch 2 oder 3, **dadurch gekennzeichnet, dass** die Pilze ausgewählt sind aus der Spezies Candida.

5. Verwendung nach Anspruch 2 oder 3, **dadurch gekennzeichnet, dass** die Pilze ausgewählt sind aus den Gattungen Aspergillus, Penicillium, Cladosporium und/oder Mucor.

6. Verwendung nach einem der Ansprüche 1 bis 5, **dadurch gekennzeichnet, dass** die nichtionischen Tenside in Träger-gebundener Form eingesetzt werden.

7. Verwendung nach Anspruch 6, **dadurch gekennzeichnet, dass** es sich bei der Träger-gebundenen Form um Kieselsäureester oder Polymerester der nichtionischen Tenside handelt.

8. Verwendung nach einem der Ansprüche 1 bis 7, **dadurch gekennzeichnet, dass** die nichtionischen Tenside nicht-kovalent an die Oberflächen gebunden werden.

9. Verwendung nach einem der Ansprüche 1 bis 8, **dadurch gekennzeichnet, dass** die nichtionischen Tenside in den eingesetzten Endkonzentrationen nicht biozid oder biostatisch wirken.

10. Verwendung von nichtionischen Tensiden zur Verminderung der Anhaftung von Mikroorganismen an Oberflächen, **dadurch gekennzeichnet, dass** die nichtionischen Tenside in die vor Adhäsion zu schützenden Materialien eingearbeitet werden oder in diese eingearbeitet sind.

11. Verwendung nach einem der Ansprüche 1 bis 10, **dadurch gekennzeichnet, dass** die nichtionischen Tenside auf die vor Adhäsion zu schützenden Materialien aufgebracht werden oder auf diese aufgebracht sind.

12. Verwendung nach einem der Ansprüche 1 bis 11, **dadurch gekennzeichnet, dass** die Anhaftung von Mikroorganismen auf Filtermedien, Baustoffen und/oder Bauhilfsstoffen vermindert wird.

13. Verwendung nach Anspruch 12, **dadurch gekennzeichnet, dass** die Baustoffe und/oder Bauhilfsstoffe ausgewählt sind aus Klebe-, Dichtungs-, Spachtel und Anstrichmassen, Kunststoffen, Lacken, Farben, Putz, Mörtel, Estrich, Beton, Isoliermaterialien und Grundierungen.

14. Verwendung nach einem der Ansprüche 1 bis 11, **dadurch gekennzeichnet, dass** die Anhaftung von Mikroorganismen auf Textilien, Keramiken, Metallen, Pelzen, Papier, Fellen, Leder und/oder Kunststoffen vermindert wird.

15. Verwendung nach einem der Ansprüche 1 bis 11 oder 14, **dadurch gekennzeichnet, dass** die Anhaftung von Mikroorganismen auf Prothesen oder Zahnersatz vermindert wird.

16. Verwendung nach einem der Ansprüche 1 bis 14, **dadurch gekennzeichnet, dass** die nichtionischen Tenside in Mitteln zur Ausrüstung von Verpackungen, Filtermedien, Klebstoffen, Baustoffen, Bauhilfsstoffen, Textilien, Pelzen, Papier, Fellen oder Leder eingesetzt werden.

17. Verwendung nach einem der Ansprüche 1 bis 14, **dadurch gekennzeichnet, dass** die Verwendung in Waschmitteln, Reinigungsmitteln, Nachspülmitteln, Handwaschmitteln, Handgeschirrspülmitteln, Maschinengeschirrspülmitteln oder Textilbehandlungsmitteln erfolgt.

18. Verwendung nach einem der Ansprüche 1 bis 11, **dadurch gekennzeichnet, dass** die Verwendung in kosmetischen Mitteln und/oder in Körperpflegemitteln erfolgt.

19. Verwendung nach Anspruch 18, **dadurch gekennzeichnet, dass** es sich bei dem kosmetischen Mittel um ein Mund-, Zahn- oder Zahnprothesepflegemittel handelt.

20. Verwendung nach Anspruch 19, **dadurch gekennzeichnet, dass** es sich bei dem Körperpflegemittel um eine Creme, Lotion, ein Dusch-, Schaum- und/oder Fußbad oder ein Haarbehandlungsmittel handelt.

21. Verwendung nach einem der Ansprüche 1 bis 20, **dadurch gekennzeichnet, dass** die nichtionischen Tenside in Mengen bis zu 20 Gew.-% enthalten sind.

22. Baustoffe und Bauhilfsstoffe ausgewählt aus Klebe-, Dichtungs- und Spachtelmassen, **dadurch gekennzeichnet, dass** sie nichtionische Tenside enthalten und/oder mit diesen ausgerüstet wurden.

23. Baustoffe und Bauhilfsstoffe nach Anspruch 22, **dadurch gekennzeichnet, dass** die nichtionischen Tenside in einer Menge bis zu 20 Gew.-% enthalten sind.

## Claims

1. The use of non-ionic surfactants for reducing adhesion of micro-organisms on surfaces, **characterized in that** the non-ionic surfactants are selected from:
a) addition products of 5 to 15 ethylene oxide units on C₆-C₁₂ alkylphenols,
b) addition products of 10 to 22 ethylene oxide units on fatty alcohols with 10 to 22 C atoms,
c) addition products of between 5 and 11 ethylene oxide units on fatty alcohols with 10 to 20 C atoms,
d) addition products of between 8 and 12 ethylene oxide units on fatty alcohols with 18 and 26 C atoms,
e) addition products of between 3 and 7 ethylene oxide units and between 2 and 6 propylene oxide units on fatty alcohols with between 10 and 16 C atoms.

2. The use according to claim 1, **characterized in that** the micro-organisms are selected from the group of bacteria and of fungi.

3. The use according to claim 2, **characterized in that** the fungi are selected from yeasts, mould fungi and/or keratinophilic fungi.

4. The use according to claim 2 or 3, **characterized in that** the fungi are selected from the *Candida* species.

5. The use according to claim 2 or 3, **characterized in that** the fungi are selected from the *Aspergillus, Penicillium, Cladosporium,* and/or *Mucor* genera.

6. The use according to any of claims 1 to 5, **characterized in that** the non-ionic surfactants are applied in a carrier-bound form.

7. The use according to claim 6, **characterized in that** as for the carrier-bound form, these are silicic acid esters or polymeric esters of non-ionic surfactants.

8. The use according to any of claims 1 to 7, **characterized in that** the non-ionic surfactants are bound in a non-covalent way to the surfaces.

9. The use according to any of claims 1 to 8, **characterized in that** the non-ionic surfactants in the final applied concentrations do not act biocidally or biostatically.

10. The use of non-ionic surfactants for reducing the adhesion of micro-organisms on surfaces, **characterized in that** the non-ionic surfactants are incorporated into the materials to be protected from adhesion or have been incorporated therein.

11. The use according to any of claims 1 to 10, **characterized in that** the non-ionic surfactants are applied on the materials to be protected from adhesion or have been applied thereon.

12. The use according to any of claims 1 to 11, **characterized in that** the adhesion of micro-organisms on filter media, building materials and/or auxiliary building materials is reduced.

13. The use according to claim 12, **characterized in that** the building materials and/or auxiliary building materials are selected from adhesive, sealing, filler and paint materials, plastics, lacquers, paints, plaster, mortar, screed, concrete, insulating materials and primary coats.

14. The use according to any of claims 1 to 11, **characterized in that** the adhesion of micro-organisms on textiles, ceramics, metals, furs, paper, fells, leather and/or plastics is reduced.

15. The use according to any of claims 1 to 11 or 14, **characterized in that** the adhesion of micro-organisms on prostheses or dentures is reduced.

16. The use according to any of claims 1 to 14, **characterized in that** the non-ionic surfactants are applied in agents for finishing packages, filter media, adhesives, building materials, auxiliary building materials, textiles, furs, paper, fells, or leather.

17. The use according to any of claims 1 to 14, **characterized in that** the use takes place in washing agents, cleaning agents, rinsing agents, hand washing agents, hand dish-washing agents, machine dish-washing agents or textile treating agents.

18. The use according to any of claims 1 to 11, **characterized in that** the use takes place in cosmetic agents and/or in body care agents.

19. The use according to claim 18, **characterized in that** as for the cosmetic agent, this is a mouth, dental, or dental prosthesis care agent.

20. The use according to claim 19, **characterized in that** as for the body care agent, this is a cream, lotion, shower, foam and/or foot bath or a hair conditioning agent.

21. The use according to any of claims 1 to 20, **characterized in that** the non-ionic surfactants are contained in amounts up to 20% by weight.

22. Building materials and auxiliary building materials selected from adhesive, sealing and filler materials, **characterized in that** they contain non-ionic surfactants and/or were provided with the latter.

23. Building materials and auxiliary building materials according to claim 22, **characterized in that** the non-ionic surfactants are contained in an amount up to 20% by weight.

## Revendications

1. Utilisation d'agents tensioactifs non ioniques pour diminuer l'adhésion de microorganismes sur des surfaces, **caractérisée en ce que** les agents tensioactifs non ioniques sont choisis parmi :
a) les produits d'addition de 5 à 15 unités d'oxyde d'éthylène sur des alkylphénols en C₆-C₁₂,
b) les produits d'addition de 10 à 22 unités d'oxyde d'éthylène sur des alcools gras comprenant 10 à 22 atomes de carbone,
c) les produits d'addition d'entre 5 et 11 unités d'oxyde d'éthylène sur des alcools gras comprenant 10 à 20 atomes de carbone,
d) les produits d'addition d'entre 8 et 12 unités d'oxyde d'éthylène sur des alcools gras comprenant 18 et 26 atomes de carbone,
e) les produits d'addition d'entre 3 et 7 unités d'oxyde d'éthylène et d'entre 2 et 6 unités d'oxyde de propylène sur des alcools gras comprenant entre 10 et 16 atomes de carbone.

2. Utilisation selon la revendication 1, **caractérisée en ce que** les microorganismes sont choisis dans le groupe des bactéries et des champignons.

3. Utilisation selon la revendication 2, **caractérisée en ce que** les champignons sont choisis parmi les levures, les champignons de pourriture et/ou les champignons kératinophiles.

4. Utilisation selon la revendication 2 ou 3, **caractérisée en ce que** les champignons sont choisis parmi l'espèce Candida.

5. Utilisation selon la revendication 2 ou 3, **caractérisée en ce que** les champignons sont choisis parmi les genres Aspergillus, Penicillium, Cladosporium et/ou Mucor.

6. Utilisation selon l'une quelconque des revendications 1 à 5, **caractérisée en ce que** les agents tensioactifs non ioniques sont utilisés sous une forme liée à un support.

7. Utilisation selon la revendication 6, **caractérisée en ce qu'**il s'agit, pour la forme liée à un support, d'esters de silice ou d'esters polymères des agents tensioactifs non ioniques.

8. Utilisation selon l'une quelconque des revendications 1 à 7, **caractérisée en ce que** les agents tensioactifs non ioniques sont liés de manière non covalente aux surfaces.

9. Utilisation selon l'une quelconque des revendications 1 à 8, **caractérisée en ce que** les agents tensioactifs non ioniques n'agissent pas de manière biocide ou biostatique aux concentrations finales utilisées.

10. Utilisation d'agents tensioactifs non ioniques pour diminuer l'adhésion de microorganismes sur des surfaces, **caractérisée en ce que** les agents tensioactifs non ioniques sont incorporés dans les matériaux à protéger contre l'adhésion ou ont été incorporés dans ceux-ci.

11. Utilisation selon l'une quelconque des revendications 1 à 10, **caractérisée en ce que** les agents tensioactifs non ioniques sont appliqués sur les matériaux à protéger contre l'adhésion ou ont été appliqués sur ceux-ci.

12. Utilisation selon l'une quelconque des revendications 1 à 11, **caractérisée en ce que** l'adhérence de microorganismes sur les agents de filtration, sur les matériaux de construction et/ou les produits auxiliaires de construction est diminuée.

13. Utilisation selon la revendication 12, **caractérisée en ce que** les matériaux de construction et/ou les produits auxiliaires de construction sont choisis parmi les adhésifs, les matériaux d'étanchéité, les enduits et les badigeons, les matériaux synthétiques, les laques, les peintures, les crépis, les mortiers, les chapes, le béton, les matériaux d'isolation et les apprêts.

14. Utilisation selon l'une quelconque des revendications 1 à 11, **caractérisée en ce que** l'adhésion des microorganismes sur les textiles, les céramiques, les métaux, les fourrures, le papier, les peaux, le cuir et/ou les matériaux synthétiques est diminuée.

15. Utilisation selon l'une quelconque des revendications 1 à 11 ou 14, **caractérisée en ce que** l'adhésion des microorganismes sur les prothèses ou les dentiers est diminuée.

16. Utilisation selon l'une quelconque des revendications 1 à 14, **caractérisée en ce que** les agents tensioactifs non ioniques sont utilisés dans des agents pour apprêter les emballages, les agents de filtration, les adhésifs, les matériaux de construction, les produits auxiliaires de construction, les textiles, les fourrures, le papier, les peaux, ou le cuir.

17. Utilisation selon l'une quelconque des revendications 1 à 14, **caractérisée en ce que** l'utilisation est réalisée dans les agents de lavage, les agents de nettoyage, les agents de rinçage, les agents de lavage à la main, les agents de lavage de vaisselle à la main, les agents de lavage de vaisselle en machine ou les agents de traitement de textiles.

18. Utilisation selon l'une quelconque des revendications 1 à 11, **caractérisée en ce que** l'utilisation est réalisée dans les agents cosmétiques et/ou les agents de soin corporels.

19. Utilisation selon la revendication 18, **caractérisée en ce qu'**il s'agit, pour l'agent cosmétique, d'un agent de soin de la bouche, des dents ou de prothèse dentaire.

20. Utilisation selon la revendication 19, **caractérisée en ce qu'**il s'agit, pour l'agent de soin corporel, d'une crème, d'une lotion, d'un bain douche, d'un bain mousse et/ou d'un bain de pieds ou d'un agent de traitement des cheveux.

21. Utilisation selon l'une quelconque des revendications 1 à 20, **caractérisée en ce que** les agents tensioactifs non ioniques sont contenus en des quantités jusqu'à 20% en poids.

22. Matériaux de construction et produits auxiliaires de construction choisis parmi les adhésifs, les masses d'étanchéité et les enduits, **caractérisés en ce qu'**ils contiennent des agents tensioactifs non ioniques et/ou **en ce qu'**ils ont été apprêtés avec ceux-ci.

23. Matériaux de construction et produits auxiliaires de construction selon la revendication 22, **caractérisés en ce que** les agents tensioactifs non ioniques sont contenus en une quantité jusqu'à 20% en poids.
